# EUROPEAN PATENT SPECIFICATION

(11) **EP 0 636 035 B1**
(45) Date of publication and mention of the grant of the patent: **31.10.2001**
(21) Application number: 93900870.2
(22) Date of filing: 07.12.1992
(51) Int. Cl.: A61M 5/14, A61M 5/152

(54) **IMPROVED FLUID DELIVERY APPARATUS**
VERBESSERTE VORRICHTUNG ZUR FLÜSSIGKEITSABGABE
DISPOSITIF D'ADMINISTRATION DE FLUIDES AMELIORE

(30) Priority: 17.04.1992 US 870269
(43) Date of publication of application: 01.02.1995
(73) Proprietor: SCIENCE INCORPORATED, Bloomington, MN 55437 (US)
(72) Inventor: KRIESEL, Marshall S., Bloomington, MN 55437 (US)
(74) Representative: Dr. Weitzel & Partner
(86) International application number: PCT/US92/10482
(87) International publication number: WO 93/20863

(56) References cited:
- WO-A-91/16100
- US-A- 4 474 575
- US-A- 4 668 231
- US-A- 4 968 301
- US-A- 4 969 873
- US-A- 4 994 031
- US-A- 5 019 047
- US-A- 5 024 657
- US-A- 5 030 203
- US-A- 5 074 844

## Description

### Background of The Invention

### Field of The Invention -

The present invention relates generally to fluid delivery devices. More particularly, the invention concerns an improved apparatus for infusing medicinal agents into an ambulatory patient at specific rates over extended periods of time.

### Discussion of The Invention

Many medicinal agents require an intravenous route for administration thus bypassing the digestive system and precluding degradation by the catalytic enzymes in the digestive tract and the liver. The use of more potent medications at elevated concentrations has also increased the need for accuracy in controlling the delivery of such drugs. The delivery device, while not an active pharmacologic agent, may enhance the activity of the drug by mediating its therapeutic effectiveness. Certain classes of new pharmacologic agents possess a very narrow range of therapeutic effectiveness, for instance, too small a dose results in no effect, while too great a dose results in toxic reaction.

In the past, prolonged infusion of fluids has generally been accomplished using gravity flow methods, which typically involve the use of intravenous administration sets and the familiar bottle suspended above the patient. Such methods are cumbersome, imprecise and require bed confinement of the patient. Periodic monitoring of the apparatus by the nurse or doctor is required to detect malfunctions of the infusion apparatus.

Devices from which liquid is expelled from a relatively thick-walled bladder by internal stresses within the distended bladder are well-known in the prior art. Such bladder, or "balloon" type, devices are described in U.S. Patent No. 3,469,578, issued to Bierman and in U.S. Patent No. 4,318,400, issued to Perry. The devices of the aforementioned patents also disclose the use of fluid flow restrictors external of the bladder for regulating the rate of fluid flow from the bladder.

The prior art bladder type infusion devices are not without drawbacks. Generally, because of the very nature of bladder or "balloon" configuration, the devices are unwieldy and are difficult and expensive to manufacture and use. Further, the devices are somewhat unreliable and their fluid discharge rates are frequently imprecise.

A much more sophisticated fluid delivery apparatus is described in U.S. Patent No. 5,019,047 issued to the present inventor. This device eliminates the bladder altogether and makes use of recently developed elastomeric film laminates which, in cooperation with a plate-like base define a fluid chamber that contains the fluid which is to be dispensed. The elastomeric film membrane laminate controllably forces fluid within the chamber into fluid flow channels provided in the base. In one form of the apparatus of the invention, a thin, planar shaped flow rate control member is strategically located within the chamber to precisely control the rate of flow of the liquid toward the fluid flow channels. The flow rate control member can be very thin and can be selected to have a very precise degree of permeability so that the rate of flow of fluid into the fluid flow channels can be controlled with great accuracy.

The apparatus of the present invention comprises an improvement upon the device described in the 5,019,047 patent by providing a novel means for mixing selected parenteral fluids with various additives such as drugs and biologically active materials which are carried by substrates of a number of different types upon which the additive or beneficial agent is immobilized or removably affixed. The additives can be placed within the fluid flow path of parenteral fluid as it is introduced into the device, or alternatively, as it flows outwardly of the device from the internally disposed fluid reservoirs.

From WO 91/16100 an apparatus for controllably intermixing two or more components in a sterile, closed environment to produce a flowable substance and then for expelling the flowable substance from the apparatus at a precisely controlled rate is known. The apparatus is particularly useful for medical applications and includes a dispenser portion with its own stored energy element provided in the form of an elastomeric membrane and a coupling mechanism for coupling a drug vial to the dispenser portion for controlled mixing a medicament contained within the drug vial with a diluent stored within the dispenser portion of the apparatus via a sterile pathway.

The devices of the present invention can be used for precisely mixing virtually any type of beneficial agent such as a drug or other pharmaceutical, with a parenteral fluid such as a diluent. Following mixing, the internal stored energy means of the device controllably dispenses the mixture which may comprise antibiotics, hormones, steroids, blood anti-clotting agents, analgesics, and like medicinal agents. Similarly, the device can be used for IV chemotherapy and can accurately deliver fluids to the patient in precisely the correct quantities and at extended microfusion rates over time.

The use of state of the art thin membranes and films permits the construction of compact, low profile, laminated structures which are easy to use and inexpensive to manufacture. When the devices of the invention are to be used with ambulatory patients they are constructed of flexible materials and are provided with a thin adhesive backing which permits the device to be conveniently self-affixed to the patient's arm or other parts of the body.

### Summary of the Invention

It is an object of the present invention to provide an apparatus for expelling fluids at a precisely controlled rate which is of a compact, low profile, laminate construction. More particularly, it is an object of the invention to provide such an apparatus which can be used for the precise infusion of pharmaceutical fluids to an ambulatory patient at controlled rates over extended periods of time.

Said object is achieved by a device according to claim 1 comprising a distendable membrane assembly which cooperates with a base to define a fluid chamber having a fluid outlet in which the distendable membrane assembly is of multilaminate construction being made up of a plurality of individual membranes or layers which cooperate to controllably urge fluid within the fluid chamber outwardly of the fluid outlet of the device.

It is another object of the invention to provide an apparatus of the aforementioned character which is highly reliable and easy-to-use by lay persons in a non-hospital environment.

Another object of the invention is to provide an apparatus which can be factory prefilled with a wide variety of medicinal fluids or one which can readily be filled in the field shortly prior to use.

Another object of the invention is to provide an infusion device in which fluids can be delivered either at a fixed rate or at variable rates and one which is operational in all altitudes and attitudes.

Still another object of the invention is to provide an apparatus of the class described which can be used to precisely mix selected parenteral fluids with a wide variety of additives such as drugs, biologically active materials and other beneficial agents and then to controllably dispense the mixture from the device.

Yet another object of the invention is to provide an apparatus as described in the preceding paragraph in which the additive can be mixed with the parenteral fluid either as it is introduced into the device or alternatively as it is expelled from the device by the internally disposed stored energy means or elastomeric laminates.

A further object of the invention is to provide a low profile, fluid delivery device of laminate construction which can be manufactured inexpensively in large volume by automated machinery.

Another object of the invention is to provide a device of the character described in which fluid is dispelled from the apparatus through either an integral infusion needle, or through a luer type connector, by a thin, distendable membrane cooperatively associated with a thin, plate-like base.

Another object of the invention is to provide an apparatus of the aforementioned character in which the distendable member is permeable to gases at least in one direction, whereby gases within the medicinal agent can be released from the fluid chamber and not injected into the patient.

Still another object of the invention is to provide an apparatus as described in the preceding paragraphs in which the rate of fluid flow from the apparatus is precisely controlled by a thin planar shaped, fluid permeable member which forms a part of the low profile, laminate construction of the apparatus.

Another object of the invention is to provide a fluid delivery device embodying an isotropic distendable membrane with a uniform modulus of elasticity which cooperates with a base to define a fluid chamber having a fluid outlet in communication with dispensing port for dispensing fluid from the device and including a flow control element disposed intermediate the fluid outlet and the dispensing port.

Still another object of the invention is to provide a device as described in the preceding paragraph in which the flow control element comprises a restriction which controllably restricts the flow of fluid between the fluid outlet and the dispensing port.

Another object of the invention is to provide a fluid delivery device of the chamber described in the preceding paragraph which includes a rate control membrane and in which the base is provided with a multiplicity of micro-channels for conducting fluid to the rate control membrane at precise rate over a predetermined active area.

A principal object of one form of the invention is to provide a novel agent formulation dispenser adopted for use with a slightly modified version of the basic fluid delivery device of the present invention for administering an aqueous solution containing selected drugs or other chemical compounds at a controlled rate.

The further objects of the invention are achieved by a device according to one of the subclaims 2-32.

### Brief Description of The Drawings

Figure 1 is a generally perspective, exploded view of one embodiment of the present invention.
Figure 1A is a fragmentary plan view taken along lines 1A-1A of Figure 3 showing the internal construction of the apparatus of Figure 30.
Figure 1B is an enlarged cross-sectional view of the circled area shown in Figure 3.
Figure 2 is a fragmentary, generally perspective view of the circled portion of the flow rate control membrane as shown in Figure 1.
Figure 3 is a cross-sectional view of the apparatus shown in Figure 1.
Figure 4 is an exploded, generally perspective view of still another form of the apparatus of the invention.
Figure 5 is a fragmentary plan view of the fluid outlet portion of the apparatus of Figure 4, partly broken away to show internal construction.
Figure 6 is a cross-sectional view taken along lines 6-6 of Figure 5.
Figure 7 is a cross-sectional view taken along lines 7-7 of Figure 5.
Figure 8 is a cross-sectional view taken along lines 8-8 of Figure 7.
Figure 9 is a fragmentary, generally perspective view of a portion of the flow control means of this form of the apparatus of the invention.
Figure 10 is a fragmentary, generally perspective view of the distendable membrane of the present form of the invention illustrating the laminate construction thereof.
Figure 11 is a plan view of another form of the invention, partly broken away to show internal construction.
Figure 12 is an exploded, generally perspective view of the form of the apparatus of the invention shown in Figure 11.
Figure 13 is a cross-sectional view taken along lines 13-13 of Figure 11.
Figure 14 is a cross-sectional view taken along lines 14-14 of Figure 13.
Figure 15 is a cross-sectional view taken along lines 15-15 of Figure 13.
Figure 16 is a generally perspective view of various forms of activating members for activating the parenteral fluid with a medicament which are usable with this embodiment of the apparatus of the invention.
Figure 17 is a fragmentary, cross-sectional view similar to Figure 15 illustrating the use of flow distribution materials within the liquid flow manifolds of the device.
Figure 18 is an exploded, generally perspective view of another embodiment of the invention.
Figure 19 is an exploded, generally perspective view of still another form of the fluid delivery apparatus of the invention.
Figure 20 is a plan view of the apparatus partially broken away to show internal construction.
Figure 21 is a cross-sectional view taken along lines 21-21 of Figure 20.
Figure 22 is a cross-sectional view taken along lines 22-22 of Figure 21.
Figure 23 is an enlarged side elevational view partly in cross-section to show internal construction of an additive carrying subassembly shown in Figure 23.
Figure 24 is an enlarged end view of one end cap of the additive subassembly shown in Figure 23.
Figure 25 is a side elevational view partly broken away of the end cap shown in Figure 24.
Figure 26 is an exploded view of the additive subassembly shown in Figure 23 partly in cross-section to better illustrate the construction of the subassembly.
Figure 27 is a fragmentary, generally perspective view of one of the end caps of the additive subassembly of the apparatus.
Figure 28 is an enlarged, cross-sectional view taken along lines 28-28 of Figure 20.
Figure 29 is a side elevational view of the apparatus of this form of the invention being filled by a hypodermic syringe of standard construction.
Figure 30 is a fragmentary, side elevational view similar to Figure 28 showing the fluid flow path of the fluid flowing through the apparatus and outwardly toward the dispensing means.
Figure 31 is a cross-sectional view taken along lines 31-31 of Figure 28.
Figure 32 is an exploded, generally perspective view of still another form of the apparatus of the invention.
Figures 32A, 32B, 32C, and 32D are general diagrammatic views illustrating various means for affinity attachment of ligands, protein molecules and enzymes to substrates.
Figure 33 is a plan view of the form of the invention shown in Figure 32.
Figure 34 is an enlarged, cross-sectional view taken along lines 34-34 of Figure 33.
Figure 35 is a cross-sectional view taken along lines 35-35 of Figure 34.
Figure 36 is an exploded, generally perspective view of yet another form of a fluid delivery apparatus not covered by the invention which includes an immobilized drug vial of unique construction that is disposed within the outlet fluid flow path.
Figure 37 is an exploded generally perspective, fragmentary view of a device similar to that shown in Figure 36, but one in which the fluid enters the device from the side rather than the end.
Figure 38 is an enlarged, cross-sectional view of the immobilized drug vial assembly usable with the devices shown in Figures 36 and 37.
Figure 39 is an end view of the immobilized drug vial assembly.
Figure 40 is an enlarged side elevational view partly in cross-section of the upper portion of the device illustrated in Figure 36 showing the immobilized drug vial or additive carrying subassembly disposed within the device.
Figure 41 is a cross-sectional view taken along lines 41-41 of Figure 40.
Figure 42 is a fragmentary, cross-sectional view illustrating the fluid flow control check valve of the device in a closed position.
Figure 43 is a generally perspective view of another embodiment of the invention which includes a novel filling needle assembly having a unique check valve housing with luer end and safety shield.
Figure 44 is an enlarged, cross-sectional view of the filling needle assembly of Figure 43.
Figure 45 is an end view of the check valve housing of this form of the invention illustrating the location of the fluid flow channels.
Figure 46 is an enlarged side view partly in cross-section showing the filling needle assembly connected with a fluid inlet line and also interconnected with the housing assembly of the device, the needle of the assembly having penetrated the housing assembly septum.
Figure 47 is a cross-sectional view similar to Figure 46 but showing the device in a filling mode, the check valve having been moved to an open position.
Figure 48 is a cross-sectional view taken along lines 48-48 of Figure 47.
Figure 49 is a cross-sectional view taken along lines 49-49 of Figure 47.

### Description of The Invention

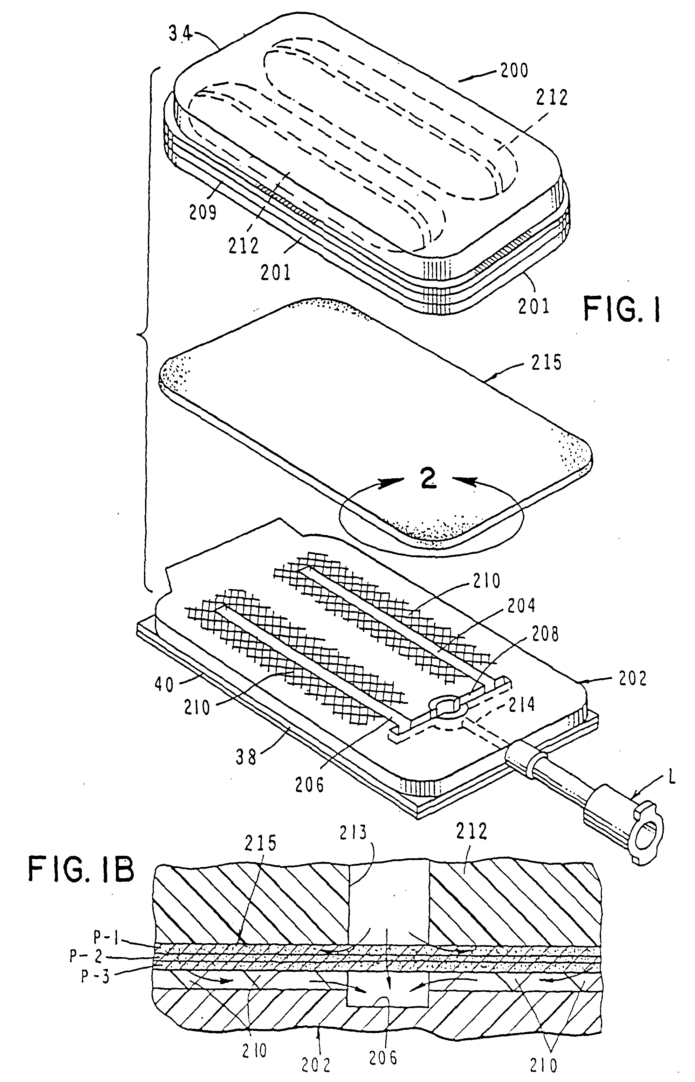

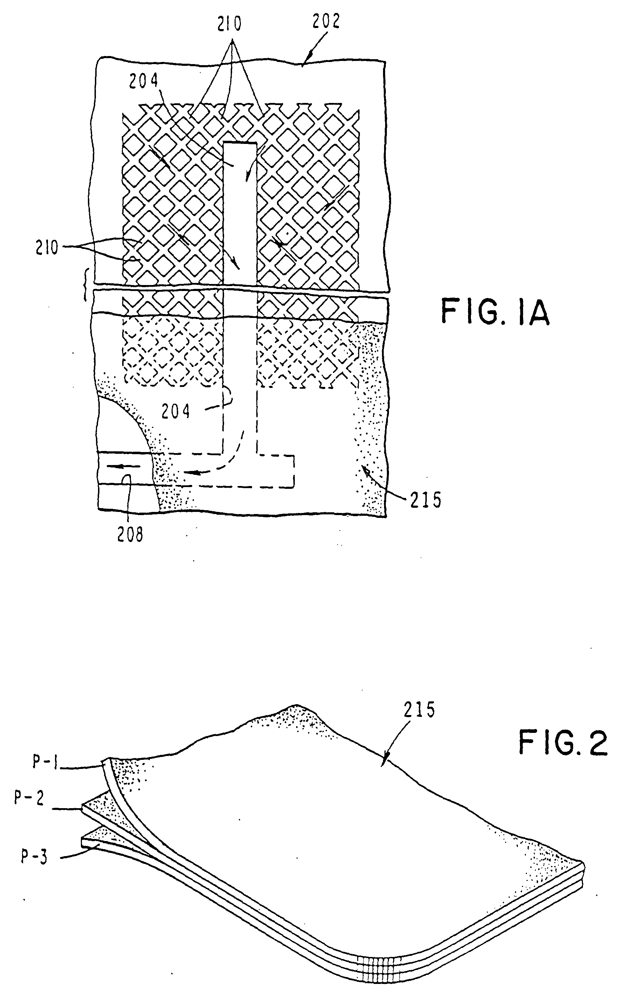

Referring to the drawings and particularly to Figures 1 through 3, one embodiment of the apparatus for use in infusing medicinal fluids into a patient is there illustrated and generally designated by the numeral 200. The apparatus of this form of the invention includes a base which comprises a pair of thin, generally planar plate-like base members 201 and 202. Base member 202 has a pair of flow rate control channels provided here as longitudinally extending fluid conduits 204 and 206. Conduits 204 and 206 are interconnected by a fluid transfer manifold, or transverse conduit 208, which, in turn, is interconnected with a fluid outlet passageway 214. Affixed to the bottom of base member 202 is a planar shaped foam pad 38. A peel strip 40 is connected to the bottom of pad 38 by any suitable means so that it can be removed to expose a layer of adhesive provided on the bottom surface of pad 38.

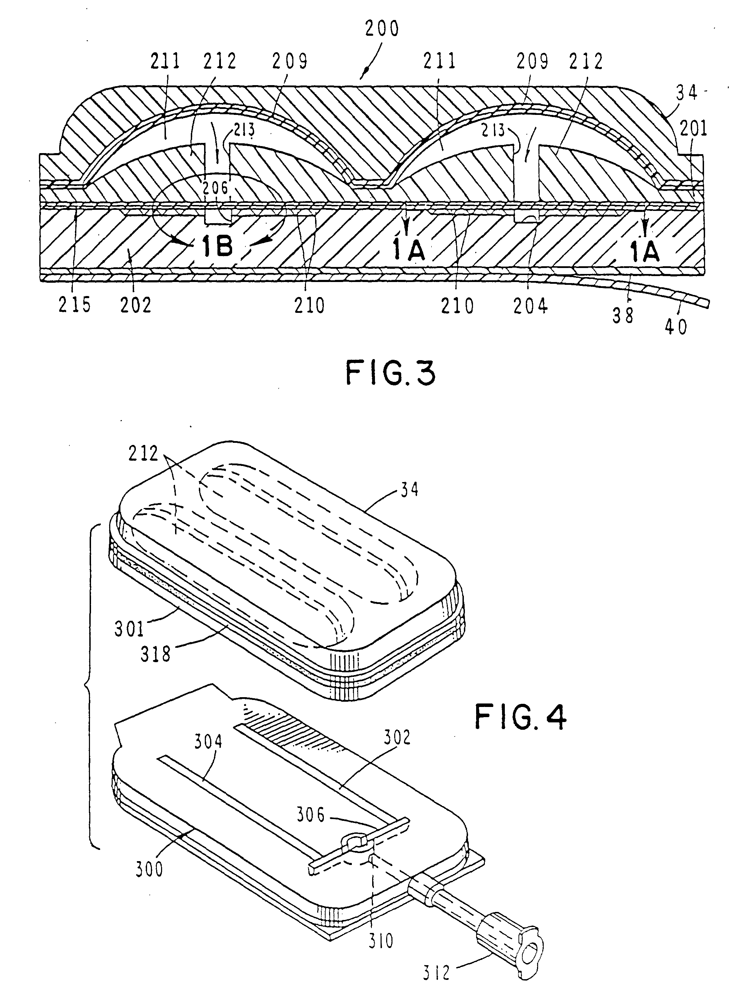

A thin, initially generally planar distendable elastomeric laminate, or assemblage 209 cooperates with base 202 to form chambers 211 (Figure 3). Assemblage 209 is distendable in the manner shown in Figure 3 by the introduction of fluid into the chambers under pressure in various manners which will presently be described. As the distendable laminate 209 is distended by the fluid pressure, internal stresses are formed therein which continuously urge it to return toward its original non-distended configuration and toward engagement with distendable membrane engagement means here provided as protuberances 212 formed on base member 201.

An important feature of this embodiment of the invention, in addition to the laminate membrane constructions, is the provision of a multiplicity of flow rate control micro-channels 210 in base 202. Micro-channels 210 are disposed on either side of conduits 204 and 206 and communicate therewith via flow control means of a character presently to be described.

In the embodiment of the invention shown in Figures 1 through 3, the flow control means is also provided in the form of a thin, laminate or gradiated assembly 215 which is superimposed over channels 210 in the manner shown in Figure 3. With this construction, when the device is in a fluid discharge mode, fluid within chambers 211 is forced by laminate 209 through channels 213 provided in protuberances 212, through assembly 215, through micro-channels 210, into conduits 204 and 206 and outwardly through outlet passageway 214. Passageway 214 is connected with dispensing means shown here as luer connection assembly L which includes a fluid flow conduit and fluid dispensing port of luer connector "L". By controlling the area of the micro-channels, the active surface area of membrane assembly 215 which is exposed to fluid can be varied in a manner to optimize fluid flow through assembly 215. Similarly by controlling the size and shape of the micro-channels, the rate of flow of the fluid through membrane assembly 215 can also be uniformly maintained.

As indicated in Figure 2, the flow control means of this form of the invention, here comprises an assemblage of a plurality of layers of permeable materials, P-1, P-2, and P-3, each having selected characteristics. These layers, which may be composites, thin films, or porous substances, may be constructed of any of the materials previously described herein so that the fluid pressure flow characteristics of the assemblage can be optimized for the particular medicinal or other fluid being dispensed. For example, layer P-1 may comprise an asymmetric membrane, or film, having a first porosity, P-2 may comprise a resin membrane or film having a second porosity and layer P-3 may comprise a carrier substrate of predetermined porosity. In another application P-1 and P-3 may take the form of carrier substrates and P-2 may comprise a very thin, rate control element. In this way a unique composite, sandwich-like assemblage can be constructed.

The multilayered, or gradient layer constructions of the stored energy source and the flow control means described in the preceding paragraphs permits easier, thin-film manufacture, precise flow control over extended periods and easier handling of the membrane film during system manufacture.

Superimposed over the assembly comprising base members 201 and 202, distended membrane 209 is a porous plastic cover assembly 34 which functions to provide an inner engagement surface for membrane 209 and a venting means for venting gases, if any, contained within the medicinal agent.

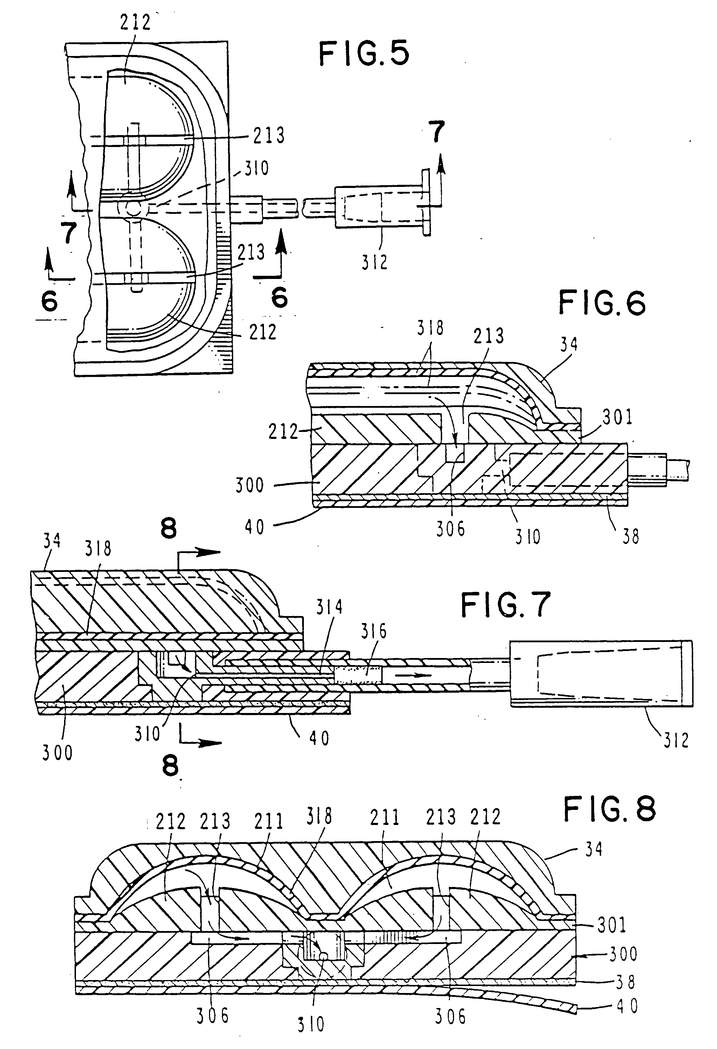

Referring now to Figures 4 through 10, still another embodiment of the present invention is there shown. This embodiment of the invention is also similar to that shown in Figures 1 through 3 and like numerals are used to identify like components. As best seen in Figure 4, the device comprises base members 300 and 301. Base member 300 has a pair of flow rate control channels 302 and 304 and a transversely extending fluid transfer manifold conduit 306. Conduit 306 is connected with an outlet 310 (Figure 5) which, in turn, is in communication with a fluid dispensing means, shown here as a fluid dispensing port 312.

Unlike the embodiment of the invention previously described, the device of this form of the invention does not include a flow rate control membrane of the character previously described. Rather, the rate of fluid flowing from the dispensing means of the device is controlled by flow control means disposed intermediate outlet 310 and fluid dispensing port 312. The flow rate control means is here provided as a fluid flow micro-conduit 314 and a porous member 316 (Figure 7) which functions to restrict the flow of fluid between outlet 310 and dispensing port 312.

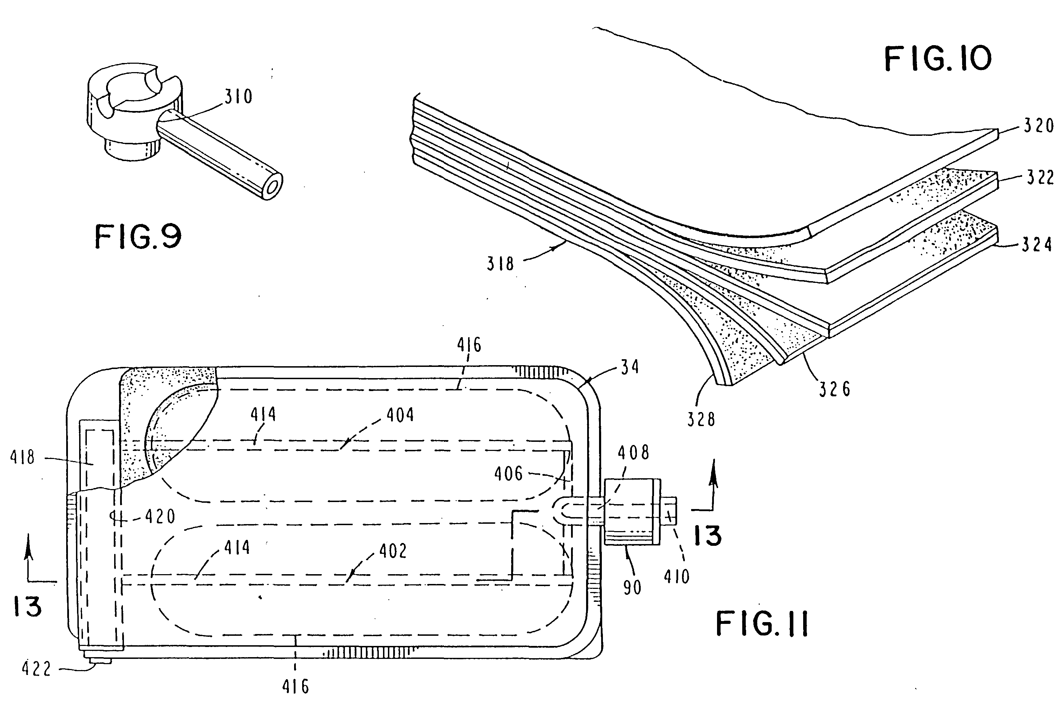

In the present embodiment of the invention stored energy means once again comprises a laminate assemblage made up of a plurality of initially generally planar distendable elements or films. Referring particularly to Figure 10, the stored energy means is there shown as a laminate assemblage 318 made up of individual elements or membranes 320, 322, 324, 326 and 328. Assemblage 318 functions in much the same way as the earlier described distendable membranes, and uniformly cooperates with base 300 to define fluid chambers or reservoirs 211. However, by constructing the stored energy means from a composite of several distinct elements or layers, the elastomeric characteristics of the stored energy means can be precisely tailored and the stored energy means can be uniquely constructed to function also as a gas permeability valve as well as the means for expelling fluids from the fluid reservoir This unique, multilayered or gradient construction permits venting to atmosphere through the membrane surface certain selected, entrained gases or vapors in the reservoir while simultaneously precluding any negative migration of selected atmospheric gases or vapors into the reservoir. The composite being made up of two or more layers of alternating thickness and permeability, and the permeability constants of the individual film layers are pressure dependent, the permeability of the stored energy means is effected and the direction of flow of the permiant through the membrane wall is controlled by the order in which the individual layers or gradiations of the composite are assembled.

For example, referring to Figure 10, layer 320 which may be distal to the reservoir comprises a thin film elastomers of a first thickness and a first permeability. On the other hand, layer 328, which may be proximal to the reservoir, comprises a thin elastomer film of a second thickness and a second permeability. Layers 322, 324 and 326 may be of further alternating thickness and permeability and, if desired, may also have different perm-select characteristics. The selective arrangement of the different films each with its own individual permeability constants in ascending order, will dictate the direction of flow of selected gases and vapors through the stored energy means.

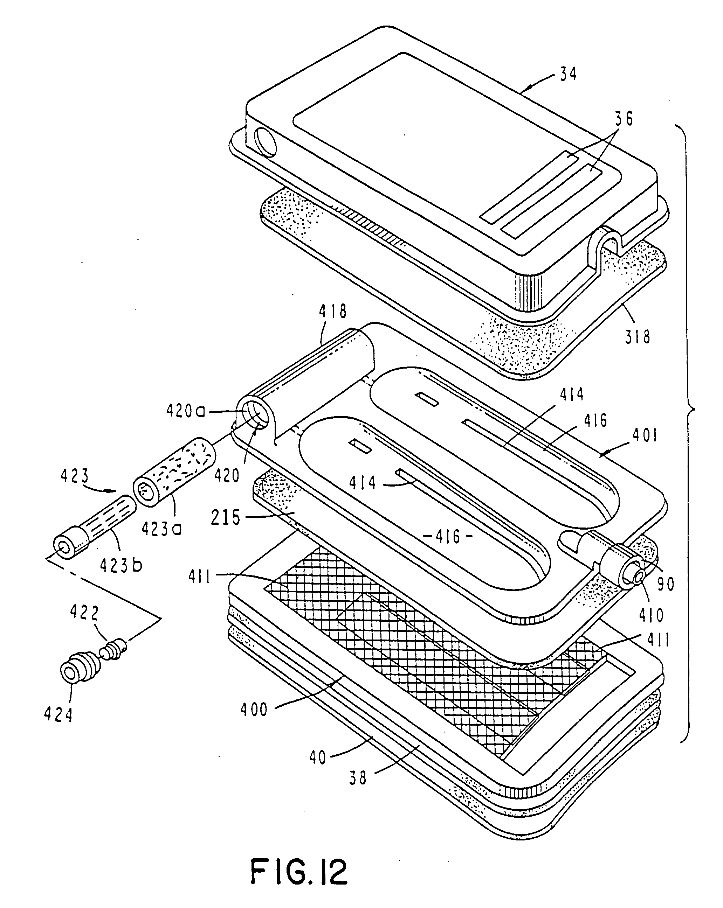

Turning now to Figures 11 through 16, another embodiment of the apparatus for use in infusing beneficial agents into a patient is there illustrated. The device of this form of the invention is unique in that it provides the opportunity to add to the diluent or other parenteral fluid being introduced into the device selected elements, chemical compounds and biologically active materials such as drugs, medicaments, biological agents, or other therapeutic agents (additives). This addition is accomplished by removably affixing the selected additives to various forms of support structures which can be placed within the path of the fluid flowing through the device so that upon contact with the fluid, the additives are released at a controlled rate to the fluid. In this way, the delivery system of the invention can be safely rendered therapeutically active upon hydration of the additive with a selected parenteral fluid such as a sterile diluent or other aqueous solvent.

The basic structure of the device of this new form of the invention is similar in many respects to the previously described embodiments of the invention and like numbers are used to identify like components.

As best seen by referring to Figure 12, the apparatus comprises a base made up of a first, generally planar member 400 and a second companion member 401 which includes a pair of longitudinally extending flow rate control conduits or channels 402 and 404 which communicate with a transversely extending fluid transfer manifold conduit 406 (Figure 11). Conduit 406 is, in turn, connected with a fluid outlet 408 which is in communication with a fluid dispensing means shown here as including a fluid dispensing port 410.

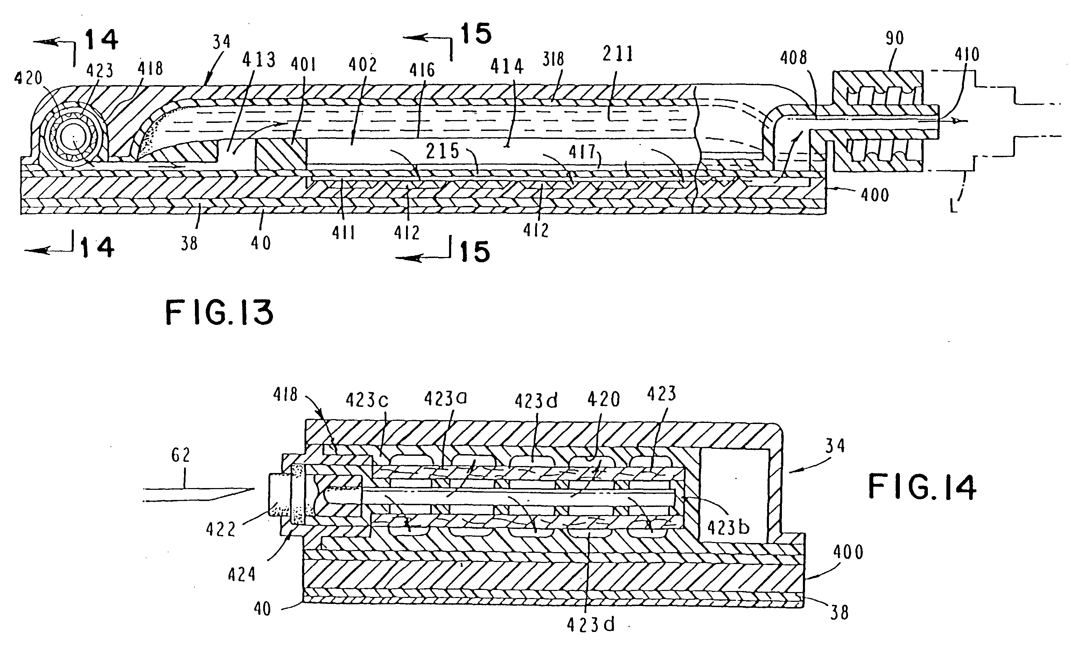

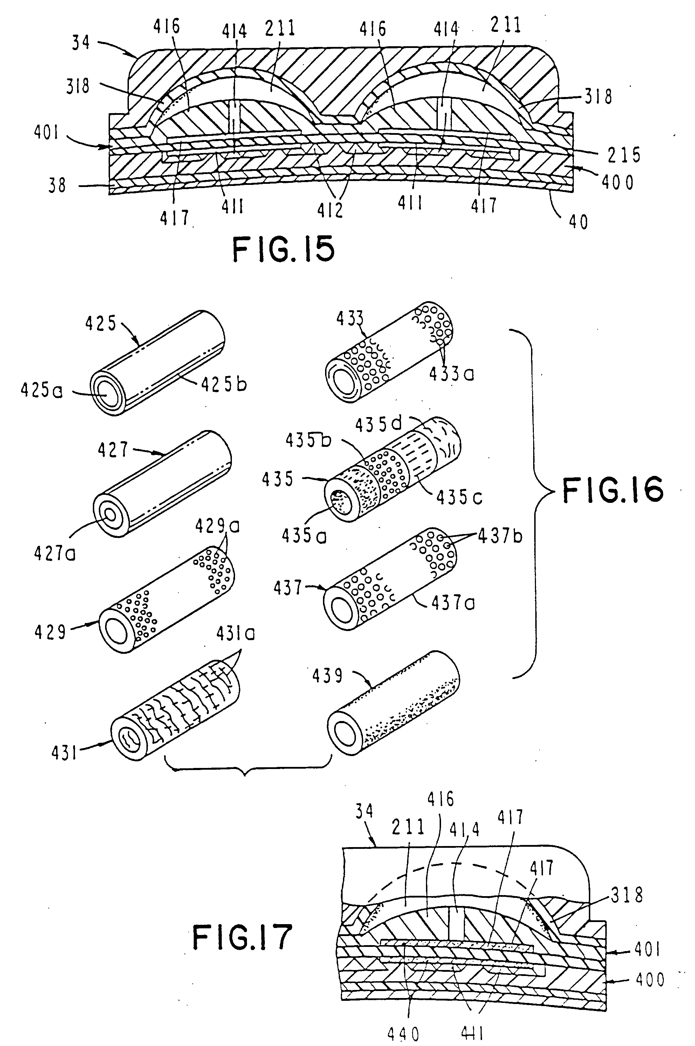

First base member 400 is also provided with a pair of longitudinally extending manifolds 411 which communicate with micro-channels 412 (Figure 15). As best seen in Figure 13, manifolds 411 and micro-channels 412 communicate with conduits 402 and 404 via flow control means of the general character described in connection with the previously described embodiment of the invention. More particularly, the flow control means is here provided as a thin, multilayered or gradiated assembly 215 (see Figure 2) which is superimposed over manifolds 411 and micro-channels 412 in the manner shown in Figure 12. Assembly 215 comprises an upper microporous layer, an intermediate rate control membrane and a lower support layer. With this construction, when the device is in a fluid discharge mode, fluid which is contained within chambers 211 (Figure 15), such as a diluent containing the additive (the beneficial agent) is initially forced by the stored energy means through channels 414 provided in a pair of upstanding protuberances 416 formed on base member 401. As the liquid passes through channels 414, it enters a pair of longitudinally extending manifolds 417 which are formed in base member 401 and which align with manifolds 411 formed in base member 400. Manifolds 417 function to uniquely distribute the fluid as it flows toward the flow control assembly 215 in a manner to disperse the fluid throughout a wide area of the flow control assembly. The uppermost layer of the assembly is designed to permit multi-axial flow distribution thereby effectively utilizing an extensive surface area of the rate control membrane. The beneficial agent flows through the rate control membrane layer, through the support layer, through micro-channels 412, and thence outwardly through outlet passageway 408. Passageway 408 in turn communicates with dispensing means, shown here as including luer connector assembly L.

As previously discussed, by controlling the area of the manifolds, the character of the uppermost layer of the flow control means, and the area of the micro-channels, the active surface area of the rate control membrane which is exposed to fluid can be precisely varied in a manner to predictably achieve the desired fluid flow control. Certain regimes of the various layers of assembly 215 can be rendered hydrophilic or hydrophobic by the appropriate use of materials and coatings in a manner and in amounts well known in the art. In this way, the wettability of the assembly can be precisely tailored as well as the initial gas venting capability of the system.

The distendable membrane engagement means, shown here as protuberances 416 (Figure 12) perform the same function as previously described. It is to be understood that in some applications only a single protuberance is provided and in other applications no protuberance at all is required.

The stored energy means of this embodiment comprises a laminate assemblage 318 made up of individual elements or membranes 320, 322, 324, 326 and 328. Assemblage 318 functions in much the same way as the earlier described, single and double layer distendable membranes, and uniformly cooperates with base member 401 to define fluid chambers or reservoirs 211 (Figure 15). However, as previously mentioned, by constructing the stored energy means from a composite of several distinct members or layers, the elastic characteristics and the resultant energy flux of the stored energy means can be precisely tailored. In this way, the stored energy means can also be uniquely constructed to function as a gas permeability valve (for example to prevent external negative migration of fluids into the reservoir) as well as the means for expelling fluids from the fluid reservoir.

As indicated in Figure 12, superimposed over the base and the stored energy source is a structural cover 34 of the character previously described having appropriate medicant and use labels 36. Affixed to the bottom of the base member 400 is a cushioning means or pad 38 having adhesive on both sides. A peel strip 40 is connected to the lower surface of pad 38. For certain applications, a thin protective film may be affixed over cover 34 to prevent ingress of liquids or other contaminants into the device.

Like the previously described forms of the invention, the present embodiment includes filling means which enables chambers 211 to be filled with a selected parenteral liquid using any suitable means such as a hypodermic syringe. To accomplish filling of the chambers, base member 401 includes an upstanding transversely extending portion 418 having a fluid passageway 420 extending therethrough. In this embodiment of the invention, the open end 420a of passageway 420 is closed by a septum means for sealably receiving a piercing element such as the hypodermic needle of a hypodermic syringe. The septum means is here provided as a needle septum 422 which is adapted to sealably close the open end 420a of passageway 420. Septum 422 is preferably constructed of a self-sealing, noncoring, puncturable material such as silicone-SEBS. It should also be understood that the septum means can also take the form of a split septum for use with a state-of-the-art blunt cannula injector system. As best seen by referring to Figure 11, passageway 420 is in communication with longitudinally extending channels 402 and 404. As before, channels 402 and 404 are, in turn, in communication with chambers 211. With this construction, an appropriate injectable such as a diluent or parenteral fluid contained within the syringe can be introduced into chambers 211 via passageway 420.

Before considering the highly important adding means of this latest embodiment of the invention a brief introductory background is perhaps helpful.

In the past it has been common practice to mix various types of separately packaged drugs with a suitable diluent immediately before they are delivered intravenously to a patient. Typically the drugs are packaged separately from the diluent for various reasons. For example, many draugs do not retain their chemical and physical stability when mixed with a diluent, and thus cannot be stored for any substantial period of time. Also, drugs are often packaged separately from the diluent because many firms which manufacture drugs are not engaged in the business of providing medical solutions in containers for intravenous delivery and vice versa.

Traditionally, the mixing of the drug and the diluent was accomplished by a doctor, nurse or medical professional injecting the injectable fluid into a glass vial containing the drug. After mixing of the drug and the diluent, the solution thus formed is withdrawn into a syringe barrel and in some instances injected immediately into the intravenous system of a patient. More typically, however, the reconstituted drug is injected from the syringe into a larger container of solution for connection to an intravenous administration set. This prior art procedure is time consuming, imprecise and generally undesirable.

The device of this latest form of the invention elegantly overcomes the drawbacks of the prior art reconstituting and delivery techniques by providing in conjunction with the basic fluid delivery device of the invention a simple and precise means for automatically mixing the desired drug with the appropriate diluent at the time the device is changed.

In the paragraphs which follow, wherein the details of this unique reconstitution process will be discussed, the following terms will have the following meanings:
Element - any of the fundamental substances that consist of atoms of only one kind and that singly or in combination constitute all matter.
Additive - the element, compound, substance, agent, biologically active material, or other material which is to be added, all or in part, to the fluid introduced into the device of the invention.
Polymer - a chemical compound or mixture of compounds formed by polymerization and consisting essentially of repeating structural units.
Parenteral Fluid - any solution which may be delivered to a patient other than by way of the intestines, including water, saline solutions, alkalizing solutions, dextrose solutions, acidifying solutions, electrolyte solutions, reagents, solvents and like aqueous solutions.
Beneficial Agents - any drug, medicament, pharmaceutical, medical polymer, enzyme, hormone, antibody, element, chemical compound or other material useful in the diagnosis, cure, mitigation, treatment or prevention of disease and for the maintenance of the good health of the patient.
Biolocrically Active Material - a substance which is biochemically, immunochemically, physiologically, or pharmaceutically active or reactive. Biologically active material includes at least one or more of the following: biochemical compounds (such as amino acids, carbohydrates, lipids, nucleic acids, proteins, and other biochemicals and substances which may complex or interact with biochemical compounds), such biochemical compounds biologically functioning as antibodies, antigenic substances, enzymes, co-factors, inhibitors, lectins, hormones, hormone producing cells, receptors, coagulation factors, growth enhancers, histones, peptides, vitamins, drugs, cell surface markers and toxins, among others known to those skilled in the art. Of the group of biologically active materials described, proteins are of utmost current interest because of the large molecule genetically engineered biopharmaceuticals as those species to be immobilized and congregated on the additive carriers hereinafter to be described. A discussion of the use of biomosaic polymers as carriers for biologically active materials is set forth in European Patent Application 0,430,517 A2.
Adding Means - an additive and any means for presenting the additive to the fluid flowing through the fluid passageways of the fluid delivery device of the invention in a manner such that all or any part of the additive will be added to the fluid. The adding means comprises the additive and the additive presentation means which may take the form of a functional support, or carrier, an anchorage, a deposition or reaction site or an element holder with or without some type of intermediate matrix or other release composition.
Additive Presentation Means - Any means such as a functional support or substrate for presenting the additive to the fluid flowing through the device. The functional substrate can comprise a polymer, copolymer, an inter-polymer, a ceramic, a crystal sponge, a carbon based matrix, a cellulosic, glass, plastic, biomosaic polymers, azlactone-functional polymer beads, adduct beads, carboxylate-functional polymer beads, gums, gels, filaments and like carriers.

By way of illustration, the adding means of the invention can take several different forms such as those illustrated in Figures 14 and 16. However, in its preferred form, the adding means includes a cylindrically shaped, functional support structure which is inserted into passageway 420 and to which various additives, including beneficial agents such as drugs, biologically active materials, and chemical elements and compounds can be releasably connected. These additives are carried by the structure in a manner such that, as the liquid flows through passageway 420 and circulates through the support assembly in the manner shown by the arrows in Figure 14, the additives will be presented to the liquid flow and efficiently added to the liquid as it flows toward chambers 211.

The additives themselves can also take various physical forms including liquid, solid, granular, powder, particle, gel, wax, hydrocolloid carrier, a gum, film, tablet, crystalline, emulsion, microcrystalline, microspherical, spray dried compounds and lyophilized compounds and saturants. The additives can be removably connected to, immobilized on, impregnated within or supported by the support means in a number of ways. The additives can be chemically or mechanically attached, affixed, or bound directly or indirectly, linked or cross linked, anchored to the surfaces of the support, or surface active agent or they can be absorbed, reaction catalyzed, electrostatically encapsulated, attached by chemical modification or transformation to the carrier surface, polymerized on or through the carrier, with or without the use of an interpolymer, localized, entrapped, suspended, deposited, impregnated, coated, or occluded or otherwise removably affixed within voids, cells, tubules, and interstices formed in the support. One important method for removably affixing the additive to the functional support means includes treating the functional support means with a compound having selected reactive functional groups such as azlactone functional compounds with their unique ability to react with aqueous media and their high binding capacity. In this way complexing agents, catalysts and biological materials such as enzymes or other proteins, as well as biomacromolecules can be attached to the carrier for later removal and recovering. Additionally, the use of one or more monomeric or polymerized surface active agents allows for rapid dissolution and smooth liberation of the additives. A discussion of such surface active agents is contained in U.S. Patent 4,963,367 issued to Ecanow.

Similarly, the additives can be added to or intermixed with the liquid flowing through the device by one or more of various mechanisms, including mechanical release, chemical reaction, dissolution, disorbsion, debinding, delinking, bioseparation, diffusion, washing, disintegration, erosion, disassociation, solubilization, leeching, enzymatic cleavage, biological reaction, osmosis, separation from ring opening materials by a ring opening reaction, and other separation means.

Additionally, a polymer can be used as the carrier or support for some component of a reaction system. Three classes of polymeric supports can be used, namely polymeric reagents, polymeric catalysts and polymeric substrates. A discussion of polymers as carriers or supports is contained in Principles of Polymerization, Second Edition by George Odian. Microporous polymers usable as carriers are also fully described in U.S. Patent 4,519,909 issued to Castro.

Turning now to Figures 12 and 14, one form of adding means, or combination additive carrier and additive is there illustrated and generally designated by the numeral 423. This form of the adding means comprises a generally cylindrically shaped assembly including a substantially cylindrical, porous substrate 423a into which an injector inlet tube 423b is closely received. Surrounding porous substrate 423a is a sleeve 423c having a multiplicity of flow channels 423d. As shown in Figure 14, the assembly thus formed is inserted into fluid passageway 420 formed in transversely extending portion 418. Receivable within the inlet opening of tube 423b is the previously identified needle septum injection site 422. Connected to portion 418, as by bonding is an insert 424 (Figure 12) which functions to contain the septum within the device.

In using the apparatus of the embodiment of the invention shown in Figures 11-15, septum injection site 422 is penetrated by a syringe needle and the sterile diluent is introduced into inlet passageway 420 using the syringe assembly. As indicated by the arrows in Figure 14, as the diluent flows longitudinally of inlet passageway 420 it will pass through porous member 423a, into flow channels 423d and then into chambers 211 urging the distendable membrane 318 outwardly into the position shown in Figures 13 and 15. As the liquid flows through porous member 423a, the additives presented to the liquid will be added to the flow, or solubilized by the diluent, thereby activating the diluent to form the therapeutic solution to be dispensed to the patient.

The liquid, such as a parenteral fluid, which is introduced into passageway 418 can include, by way of example, a reagent, a sterile diluent, various electrolytes, aqueous solutions such as aqueous solutions of dextrose, saline solutions, alkalinizing solutions, acidifying solutions, polyonic solutions and any other liquids that can serve as a vehicle for the administration of therapeutic or beneficial agents which are desirable to administer to the patient by infusion.

Turning now to Figure 16, various other forms of adding means are there illustrated. For example, numeral 425 identifies an assembly comprising a porous substrate with interconnecting voids, such as a crystal sponge 425a, over which various outer coatings 425b exhibiting one or more additives are laminated. The selected additives such as elements, chemical compounds, drugs and functional intermediates are provided on or within the coating layers by techniques well known to those skilled in the art. The additives exhibited by layers are, of course, introduced into the sterile diluent as the diluent flows along of the inlet passageway 420. In this instance, since the substrate will not be damaged by the syringe, as might a polymer or cellulosic carrier, use of the injector inlet tube 423b is not required.

Another form of additive assembly designated in Figure 16 by the numeral 427, comprises a solid tubular member having an internal, axially extending fluid passageway 427a, the inner wall of which is lined with a separation coating affixing the additives such as chemical compounds and beneficial agents, or medicaments.

Still another form of additive assembly is identified in Figure 16 by the numeral 429. This assembly comprises a cylindrical, porous plug like member made up of a multiplicity of fused together microspheres or beads 429a, each of which is coated with a separation or reactive coating upon which is deposited an additive such as a biologically active material or other beneficial agent. The microspheres can be formed of glass, plastic or other suitable materials.

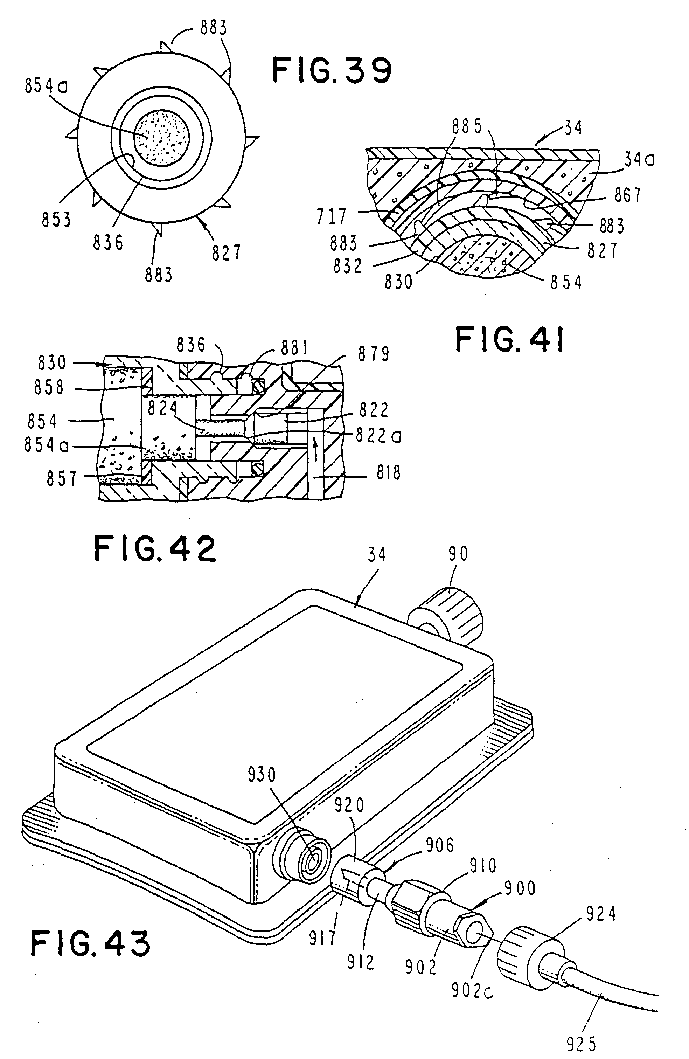

The numeral 431 of Figure 16 identifies yet another form of the adding means of the invention. In this form of the invention a generally cylindrically shaped functional support means for affinity attachment, and subsequent release of the additive, is formed from a multiplicity of microporous polymers 431a presenting a multiplicity of reactive sites over a wide area for species immobilization. In this form of the invention, in order to avoid needle damage to the polymers, it is necessary to use an injector inlet tube of the character shown in Figure 41 and identified by the numeral 423b.

The additive assembly designated in Figure 16 by the numeral 433 may also require the use of an injector tube. This assembly is made up of high porosity, semi-synthetic cellulosics 433a formed into a generally cylindrical shape and having interconnecting, interstial surfaces or functional support means and is similar in size and configuration to activating assembly 423.

Another slightly more complex additive assembly is identified by the numeral 435. This assembly is made up of a plurality of spaced apart, porous disk shaped wafers 435a, 435b, 435c, and 435d each wafer being of the same or different construction and porosity and each having reactive sites presenting to the liquid flow specially selected additives such as beneficial agents, elements or compounds so that multiple reactivities and selectivities can be achieved. With this construction, a wide variety of liquid flow rates, and complex sequential separations and priority staged substance introduction into the system reservoir can be achieved by specially designing each of the wafers that cooperate to make up the structural support.

Still another form of additive assembly is designated in Figure 16 by the numeral 437. This assembly comprises a cylindrically shaped porous structure 437a which is provided with pores of varying sizes only some of which are coated, plugged or impregnated with selected additives 437b and, as necessary, functional intermediate materials.

Finally, the functional support member identified by the numeral 439 exemplifies yet another form of adding means of the invention. This member, which is also of a generally cylindrically shaped configuration, is constructed from a porous ceramic material into which selected additives and intermediate compounds have been removably affixed. Member 439, being made of a hard ceramic, would not be easily damaged by the needle 62 and therefore the injector inlet tube is generally not needed. Member 439 can also be constructed from fuzed activated carbon particles, coated porous zirconium oxide bonded spherules, or other porous forms of polymer reactive supports including joined azlactone-functional polymer beads suitable for the attachment of functional materials.

Assemblies 423 through 439 which may be soluble or insoluble are intended to merely exemplify, not to limit, the wide variety of materials and constructions that can be used to introduce the desired additives into the liquid flow introduced into the inlet flow passageway 420 of the device.

Turning now to Figure 17, another form of the invention is shown. This figure shows in cross-section one set of the manifolds 411 and 417 which are formed in base members 400 and 401 of the device. In certain applications, it is sometimes advantageous to provide liquid distribution means within manifolds 411 and 417 to enhance the uniform flow of liquid through the manifolds. In the embodiment of the invention shown in Figure 17. the liquid distribution means is provided as generally planar strips of felt-like hydrophilic polymer material 440 which is readily wetted by the solution being expelled from chambers 211 by the stored energy means, or distendable membrane 318. Material 440 can be of any type that will enhance the uniform flow of the solution contained within chambers 211 and can include ceramic, crystalline, polymer, sponge and other similar materials which have hydrophilic characteristics.

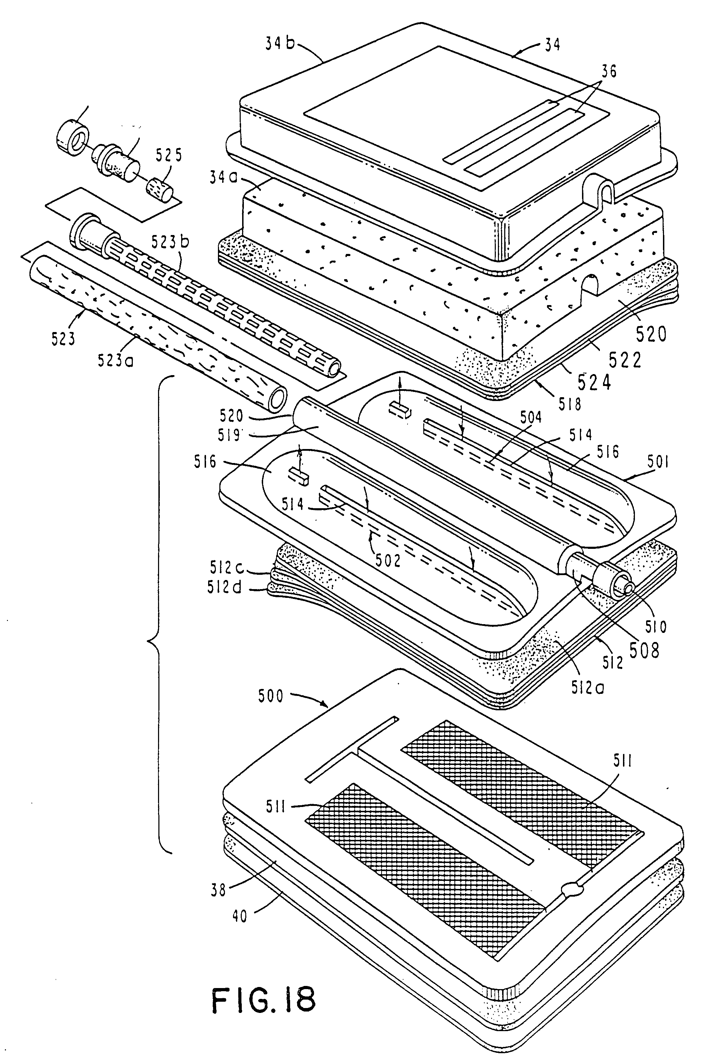

Turning to Figure 18, another embodiment of the apparatus for use in infusing beneficial agents into a patient is there illustrated. The basic structure of the device of this latest form of the invention is similar in many respects to that shown in Figure 12 and like numbers are used to identify like components. The major difference between the device shown in Figure 18 and that shown in Figure 12 is that the transverse filling portion 418 is replaced by a longitudinally extending filling portion 518. As will be discussed in greater detail hereinafter, the adding means including the additive carriers of this form of the invention are also of a somewhat different configuration.

As in the apparatus of Figure 12, the apparatus here comprises a base made up of a first, generally planar member 500 and a second companion member 501 which includes a pair of longitudinally extending flow rate control conduits or channels 502 and 504 which communicate with a fluid outlet 508 which is in communication with a fluid dispensing means shown here as including a fluid dispensing port 510.

First base member 500 is also provided with a pair of longitudinally extending manifolds 511 which communicate with micro-channels of the character shown in Figure 15 and identified by the numeral 412. Manifolds 511 as well as the micro-channels communicate with conduits 502 and 504 via flow control means. The flow control means is here provided as a thin, multilayered or gradiated assembly 512 which is superimposed over manifolds 511 in the manner indicated in Figure 18. Assembly 512 comprises an upper microporous substrate 512a, an intermediate rate control membrane 512c and a lower support substrate 512d. With this construction, when the device is in a fluid discharge mode, fluid which is contained within chambers 211 (Figure 15), such as a diluent containing the additive (the beneficial agent) is initially forced by the stored energy means through channels 514 provided in a pair of upstanding protuberances 516 formed on base member 501.

As previously discussed, by controlling the area of the manifolds, the character of the uppermost layers of the flow control means, and the area of the micro-channels, the active surface area of the rate control membrane which is exposed to fluid can be precisely varied in a manner to predictably achieve the desired fluid flow rate control. As before, certain regimes of the various layers of assembly 512 can be rendered hydrophilic or hydrophobic by the appropriate use of materials and coatings in a manner, amount, area and location well known in the art. In this way, the wettability of the assembly can be preciselv tailored as well as the initial gas venting capability of the system.

The distendable membrane engagement means, shown here as protuberances 516 perform the same function as previously described. It is to be understood that in some applications only a single protuberance is provided and in other applications no protuberance at all is required.

The stored energy means of this embodiment is also of the same general character as previously described and comprises a laminate assemblage 518 made up of individual elements or membranes 520, 522, and 524. Assemblage 518 functions in much the same way as the earlier described, single layer distendable membranes, and uniformly cooperates with base member 501 to define fluid chambers or reservoirs 211 (Figure 15). However, as previously mentioned, by constructing the stored energy means from a composite of several distinct members or layers, the elastic characteristics and the resultant energy flux of the stored energy means can be precisely tailored. As before, the stored energy means can also be uniquely constructed to function as a gas permeability valve to control gas flow in one direction as well as the means for expelling fluids from the fluid reservoir.

As indicated in Figure 18, superimposed over the base and the stored energy source is a cover 34 which includes a porous structural member 34a and film cover 34b. Appropriate medicant primary labels and instruction for use labels 36 are affixed to cover 34. Affixed to the bottom of base member 500 is a cushioning means or pad 38 having adhesive on both sides. A peel strip 40 is connected to the lower surface of pad 38.

The present embodiment also includes filling means which enables chambers 211 to be filled with a selected injectable such as a parenteral liquid using a hypodermic syringe and needle of a character well known in art. To accomplish filling of the chambers, base member 501 includes the previously mentioned upstanding longitudinally extending portion 519 having a fluid passageway extending therethrough. In this latest embodiment of the invention, the open end of the passageway is closed by a septum means for sealably receiving a piercing element such as a hypodermic needle. The septum means is here provided as a needle septum which is adapted to sealably close the open end of the passageway. Passageway is in communication with the fluid chambers 211 so that an appropriate parenteral fluid contained within a syringe assembly can be introduced into chambers 211 via passageway.

One form of adding means and additive carrier of this latest embodiment is generally designated in Figure 18 by the numeral 523. This form of the adding means comprises an elongated, generally cylindrically shaped assembly including a substantial cylindrical, porous substrate 523a into which an injector inlet tube 523b is closely received. The assembly comprising substrate 523a and injector inlet tube 523b is closely receivable within longitudinally extending fluid passageway 520 formed in longitudinally extending portion 519. Receivable within the inlet opening of tube 523b is the previously identified needle septum which is held in position by insert 524 which is bonded to base member 501.

Disposed intermediate septum and injector inlet tube 523b is a porous flow restrictor 525 which controllably resists the fluid which is flowing into the injector tube. This resistor can be constructed from any suitable porous inert material such as a ceramic or plastic which resists fluid flow in a manner to controllably regulate the residence time of the fluid being introduced into the carrier 523.

As was the case with the embodiment of the invention shown in Figures 1 through 16, various additives, including beneficial agents such as drugs, biologically active materials, and chemical elements and compounds can be releasably connected to additive carrier 523. These additives are carried by the structure in a manner such that, as the liquid flows through passageway, the additives will be presented to the liquid flow, separated and released and efficiently added to the liquid as it flows toward the reservoir chambers 211.

As before, the additives can take the various physical forms previously described herein and can be removably or releasably connected to the carrier in the many way previously described. Similarly, the additive can be added to the fluid introduced into the device by the various chemical and mechanical means previously described for easy removal and recovery.

Various other forms of adding means and additive assemblies of the character illustrated in Figure 16 can also be used with this latest embodiment. For example, a porous substrate with interconnecting voids, such as a crystal sponge 425a over which various outer coatings 425b of one or more additives are laminated can be used. Similarly, a solid tubular member such as member 427 (Figure 16) having an internal, axially extending fluid passageway 427a, the inner wall of which is lined with a separation coating or surface active agent or intermediate matrix affixing the additives such as medicaments, drugs and other beneficial agents can be used.

Still another form of additive assembly which can be used is one of the character identified in Figure 16 by the numeral 429. This assembly comprises a cylindrical member made up of a multiplicity of fused together microspheres 429a, each of which is coated with a surface active agent separation coating upon which is deposited and chemically anchored a biologically active material or other beneficial agent. Other forms of the additive means which can be used include those shown in Figure 16 and identified by the numerals 431, 433, 435, 437, and 439.

In certain applications the additive, as defined herein, can be deposited, coated or otherwise removably affixed interstitially of or onto a surface of the membrane 524 of the stored energy means which is exposed to the liquid. With this construction, the fluid entering chambers 211 will be exposed to the additive and the additive will be added to the fluid prior to its infusion into the patient.

Additionally in some applications, the additive can be removably affixed to, impregnated within or otherwise anchored directly or indirectly on or within either or both of the base members 500 and 501. In this way, the additive can be separated from the base members and added to the liquid which contacts the base members at any time prior to its infusion into the patient.

In other applications, the additive can be deposited, coated or otherwise removably affixed interstitially of or onto the rate control assembly 512 so that the additive is exposed to the fluids flowing through the rate control assembly in a direction toward the outlet of the device.

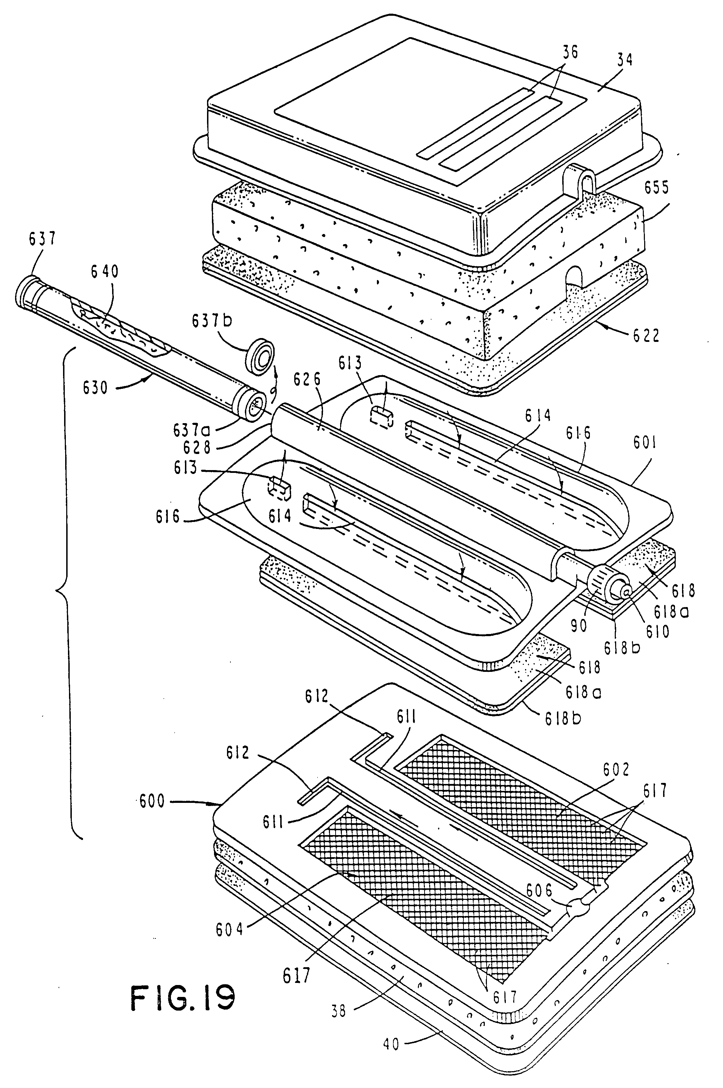

Referring to Figures 19 through 31, another embodiment of the apparatus for use in infusing beneficial agents into a patient is there illustrated. The device of this form of the invention is similar in many respects to the embodiment shown in Figures 11 through 16 in that it provides the opportunity to add to the diluent or other parenteral fluid being introduced into the device selected elements, chemical compounds and biologically active materials such as drugs, medicaments, biological agents, or otherwise therapeutic agents (additives). However, in this latest form of the invention selected additives are removably affixed to various forms of support structures which are, in turn, contained within a novel additive subassembly that can be inserted into the device in a manner to place the additives within the path of the fluid flowing through the device. In this way, the additives, such as drugs, can remain sealed in the prepackaged additive subassembly, which preferably comprises a glass vial, until such time as the subassembly is interconnected with the base of the apparatus and diluent flow through the subassembly is commenced.

Because the basic structure of the device in this latest form of the invention is similar in many respects to that previously described herein, like numbers are used to identify like components.

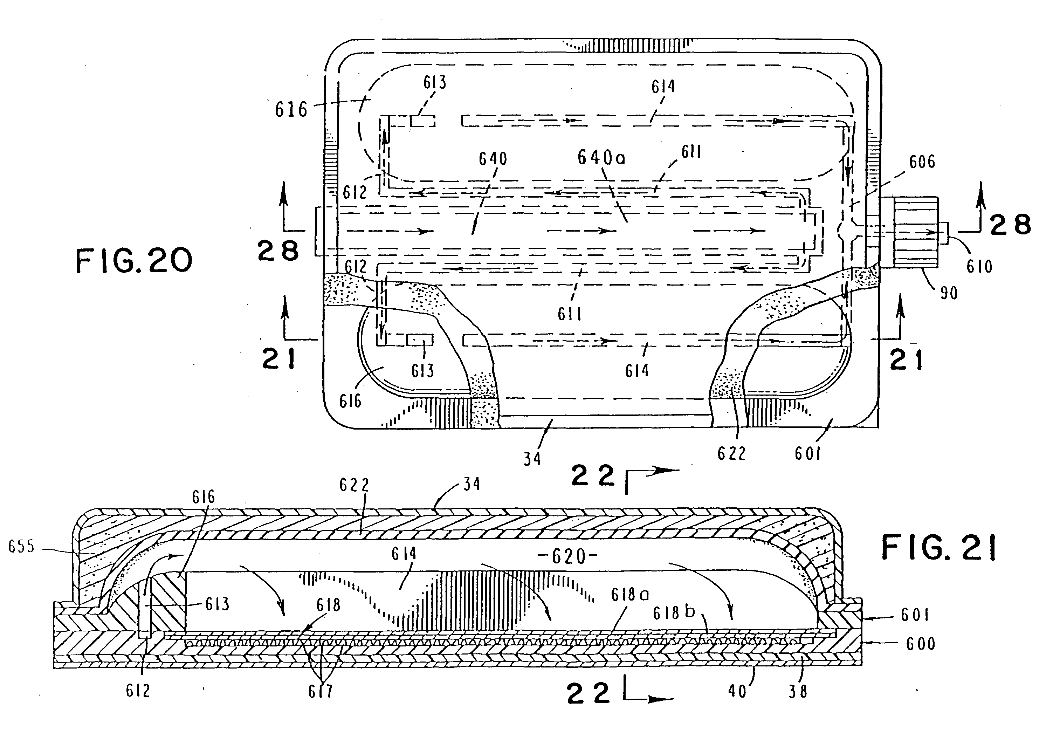

As best seen by referring to Figure 19, the apparatus comprises a base made up of a first, generally planar member 600 and a second companion member 601. Member 600 includes a pair of a longitudinally extending fluid flow manifolds 602 and 604 which communicate with a transversely extending fluid transfer passageway 606. Passageway 606 is, in turn, connected with a fluid outlet 608 which is in communication with a fluid dispensing means shown here as including a fluid dispensing port 610 (Figure 20).

First base member 600 is also provided with a pair of longitudinally extending flow passageways 611 the purpose for which will presently be described. Passageways 611 communicate with a pair of transverse channels 612 (Figure 19). Channels 612, in turn, communicate with reservoir inlet ports 613 of base member 601 which are of the character best seen in Figure 19.

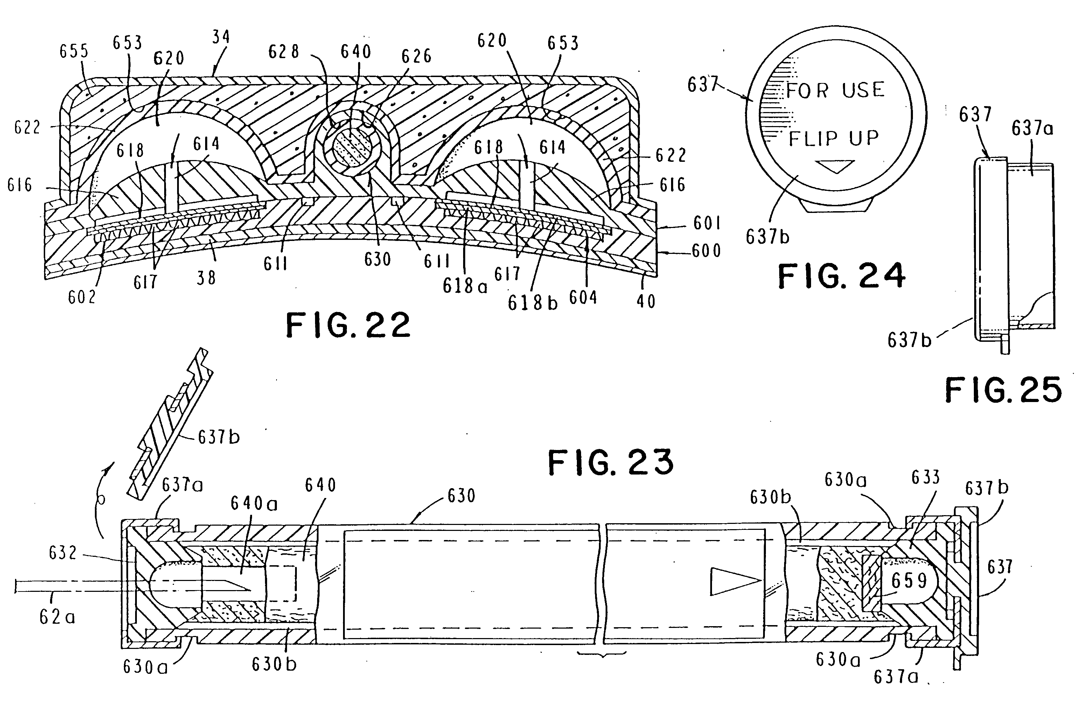

Reservoir outlet passageways for fluid flowing outwardly from the reservoir are provided in the form of longitudinally extending flow channel 614 provided in a pair of protuberances 616 which are integrally formed with base member 601. Channels 614, in turn, communicate with microchannels 617 of manifolds 602 and 604 via flow control means, shown here as membrane assemblies 618 (Figure 19) which are superimposed over manifolds 602 and 604 and micro-channels 617 in the manner shown in figure 19 and 22. Assemblies 618 comprise an upper microporous layer 618a and a lower support layer 618b. With this construction, when the device is in a fluid discharge mode, fluid which is contained within reservoirs or chamber 620 (Figure 22), such as a diluent containing the additive (the beneficial agent), is initially forced by the stored energy means through channels 614 provided in upstanding protuberances 616 formed on base member 601. As the liquid passes through channels 614, it flows through flow control assemblies 618 and into manifolds 602 and 604. After passing through the flow rate control means, the beneficial agent flows through the microchannels 617, and thence outwardly through outlet passageway 606. 606, in turn, communicates with dispensing means, shown here as including a connector assembly 90.

The distendable membrane engagement means of protuberances 616 perform the same function as previously described as does the unique stored energy means. The stored energy means of this embodiment is of the general character previously described and comprises a laminate assemblage 622 made up of at least two individual elements or membranes. As before, assemblage 622 cooperates with base member 601 to define the fluid chambers or reservoirs 620 (Figure 22).

As indicated in Figure 19, superimposed over the base and the stored energy source is a structural cover 34 of the character previously described having appropriate medicant and medicant use instruction labels 36. Affixed to the bottom of base member 600 is a cushioning means or pad 38 having adhesive on both sides. A peel strip 40 is connected to the lower surface of pad 38. For certain applications, a thin protective film may be affixed over cover 34 to prevent ingress of liquids or other contaminants into the device.

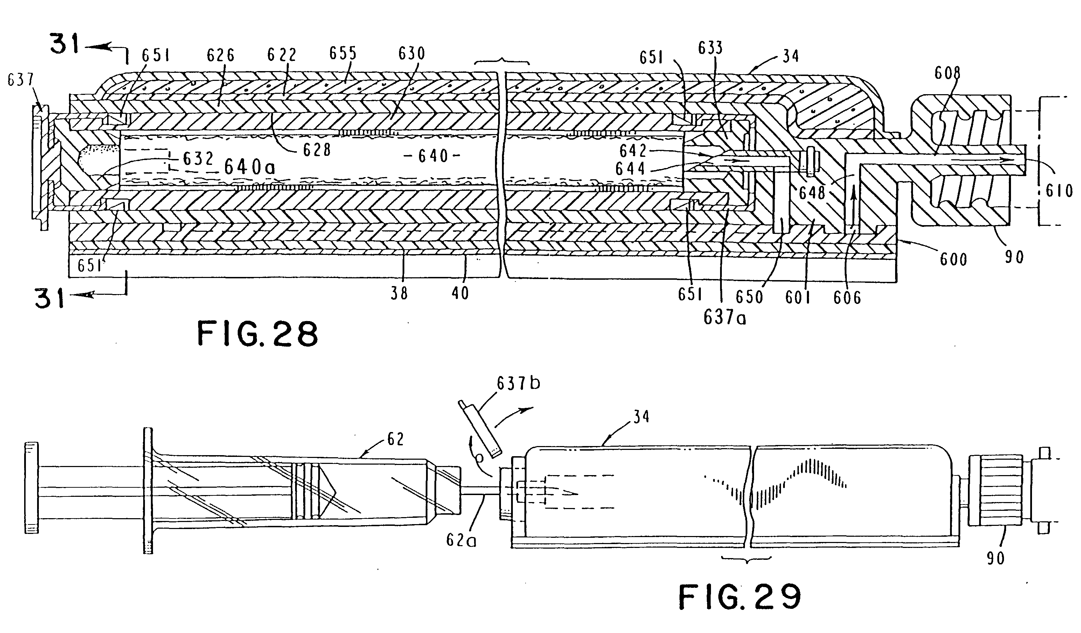

Like the previously described embodiments, the present embodiment includes filling means which enables chambers 620 to be filled with a selected parenteral liquid. Here the filling means includes a hypodermic syringe and needle of the character identified in Figure 29 by the numeral 62.

As best seen in Figures 19 and 20, base member 601 includes an upstanding, longitudinally extending portion 626 having generally cylindrical chamber 628 extending therethrough (Figure 28). In this embodiment of the invention, chamber 628 is uniquely designed to closely receive the additive subassembly that sealably contains the additive to be added to the parenteral fluid.

Referring to Figure 23, this unique immobilized adding means or additive subassembly comprises a glass tube or vial 630 which is sealed at either end by sealing means shown here as rubber stoppers 632 and 633. Stopper 632 is adapted to sealably receive a piercing element such as the hypodermic needle 62a of syringe assembly 62. (Figures 23 and 24). The rubber stoppers 632 and 633 are preferably constructed of a self-sealing, noncoring, puncturable material such as siliconeSEBS. Tear open type aluminum sealing caps 637 are provided at each end of tube 630 and function to sealably encapsulate rubber stoppers 632 and 633.

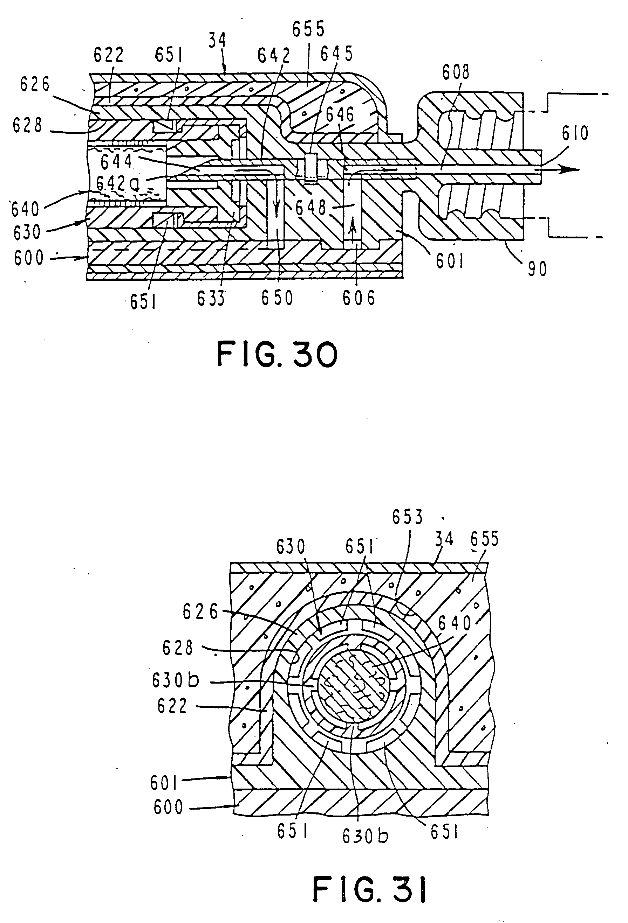

Sealing caps 637 comprise an aluminum sleeve portion 637a which is crimped in place within a groove 630a provided at either end of tube or vial 630, and a removable end plate 637b. Disposed interiorly of tube 630 is a substrate 640 which releasably carries the additive such as a beneficial agent of the character earlier defined herein (Figures 23, 26, and 27). Substrate 640 which, along with the additive, comprises the adding means of this form of the invention can be of the same character as the additive carriers of the assemblies shown in Figure 16 and identified by the numerals 425, 427, 429, 430, 433, 437, and 439. As indicated in Figure 31, vial 630 is provided with circumferentially spaced ribs 630b which maintain the substrate spaced apart from the interior wall of the vial. Such stand-off ribs may not be required when substrates of certain character are used.

Turning particularly to Figure 30, it can be seen that a uniquely configured needle 642 is integrally molded into base member 601. Needle 642 is provided with first and second fluid passageways 644 and 646 which are separated by a center section 645. Passageway 644 is in communication at one of its ends with passageway 650 of base member 601. At its opposite end, passageway 644 communicates with the interior of tube 630 after the needle point 642a has pierced rubber stopper 633 in a manner shown in Figure 30. Passageway 646 is in communication at one of its ends with a passageway 648 of the base member 601 and at its opposite end communicates with outlet passageway 608 of base member 601.

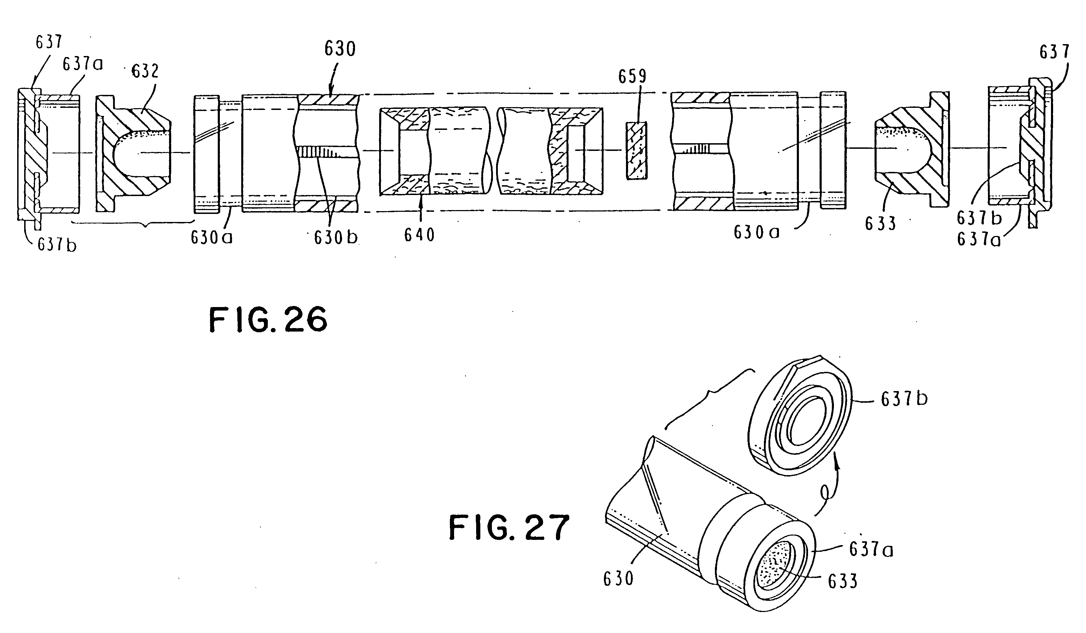

In using the apparatus of the embodiment of the invention shown in Figures 19 through 30, sealing caps 637 are opened in the manner shown in Figures 23 and 27 to expose the site injection portion of the rubber stoppers. The adding means, or glass vial 630 is then inserted into chamber 628 and urged forwardly with sufficient force to cause needle 642a to penetrate rubber stopper 633 in the manner shown in Figures 28 and 30. As best seen in Figure 28, vial locking means, here shown as detents 651, are provided at either end of chamber 628. These detents permit passage of vial 630 in an inward direction but function to lock the vial in place to prevent its withdrawal from the chamber.

With the vial locked in place within chamber 628, rubber stopper 632 is pierced by needle 62a of the needle syringe and the sterile diluent is introduced into the interior passageway 640a of substrate 640. As indicated by the arrows in Figures 20 and 30, as the diluent flows longitudinally of the additive presentation means, or substrate 640, it efficiently intermixes with the additive carried by the substrate. The mixture thus formed flows through passageway 644 of needle 642, into passageway 650 (Figure 30), through passageways 611 and 612 (Figure 20) and then into chambers 620 via ports 613. This fluid flow urges membranes 622 outwardly into the position shown in Figure 22 in close proximity with interior walls 653 of porous body 655.

As before, the liquid, such as a parenteral fluid, which is introduced into passageway 640a can include, by way of example, a reagent, a sterile diluent, various electrolytes and various other aqueous solutions.

After the additive carried by substrate 640 has been separate and released by the parenteral fluid and has been efficiently intermixed therewith, the solution or beneficial agent thus formed will remain within reservoirs 620 until the time it is to be infused into the patient. When the outlet port 610 is opened for fluid flow to the patient, the fluid contained within chambers 620 will flow downwardly through channels 614 in protuberances 616, (Figure 21), through the flow rate control membranes 618 and into crossing microchannels 617. As best seen in Figure 19 manifolds 602 and 604 communicate with transverse manifold passageways 606 which, in turn, are in communication with the fluid outlet port 610 of the device. A hydrophilic porous flow filter 659 is disposed proximate the outlet end of substrate 640 (Figure 26) for filtering and controlling the residence time of the fluid within the substrate 640.

It is to be appreciated that a wide variety of additives, such as drugs and the like, can be removably affixed to substrates of widely varying material composition and design. The substrates can then be safely sealably contained within the sealed glass vial of the additive subassembly for use at any later time with stored energy infusion devices of the character shown in Figure 19. This unique approach provides a completely new dimension to the preparation, packaging and controlled administration of virtually any kind of beneficial agent to an ambulatory patient.

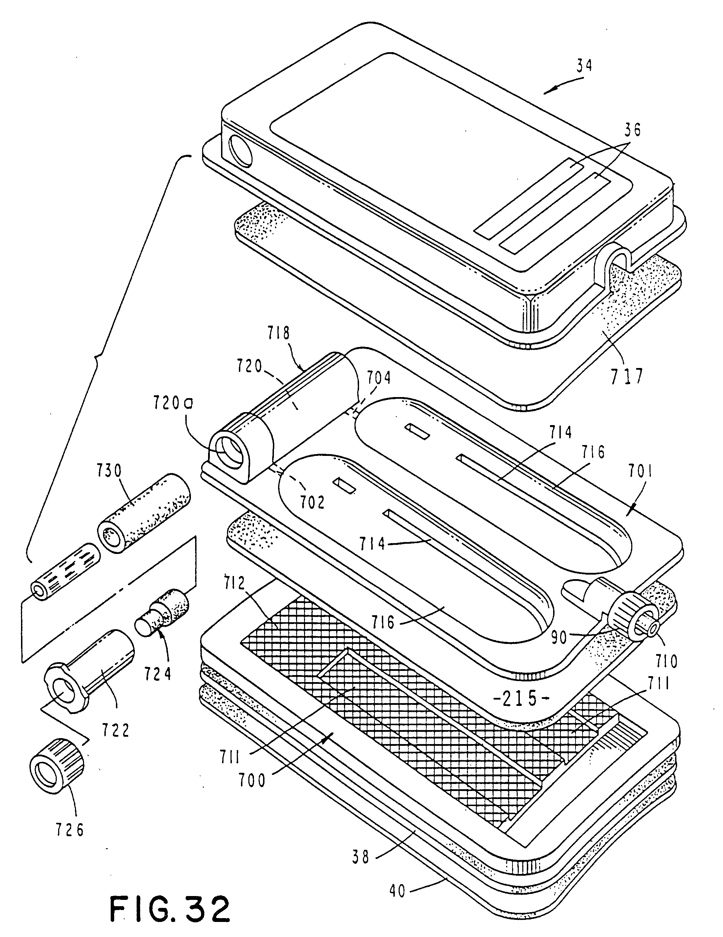

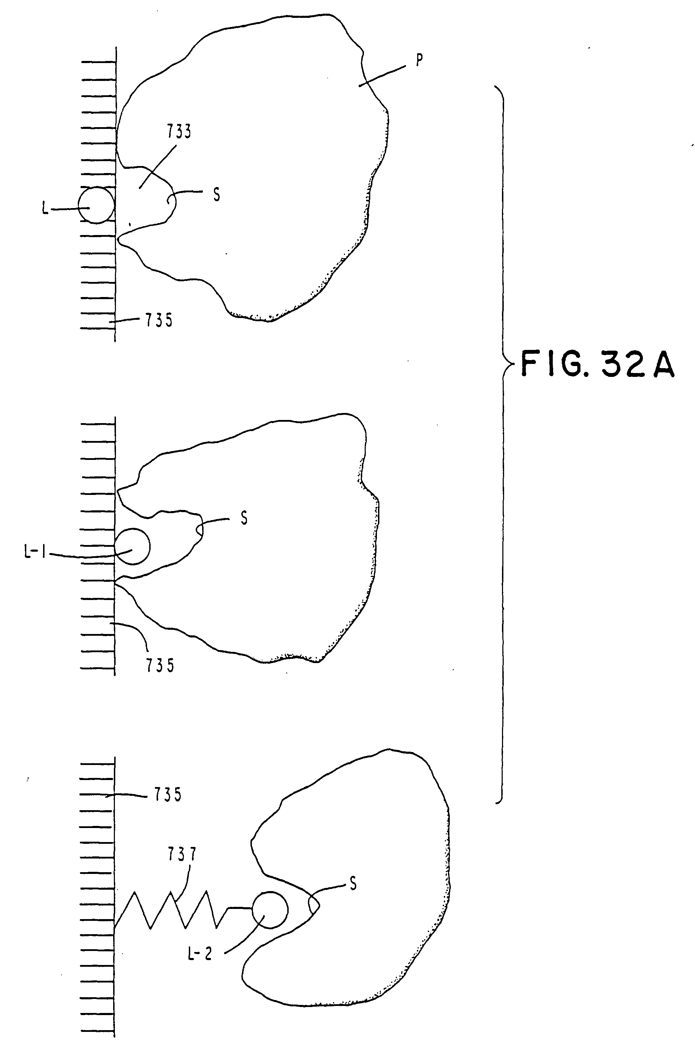

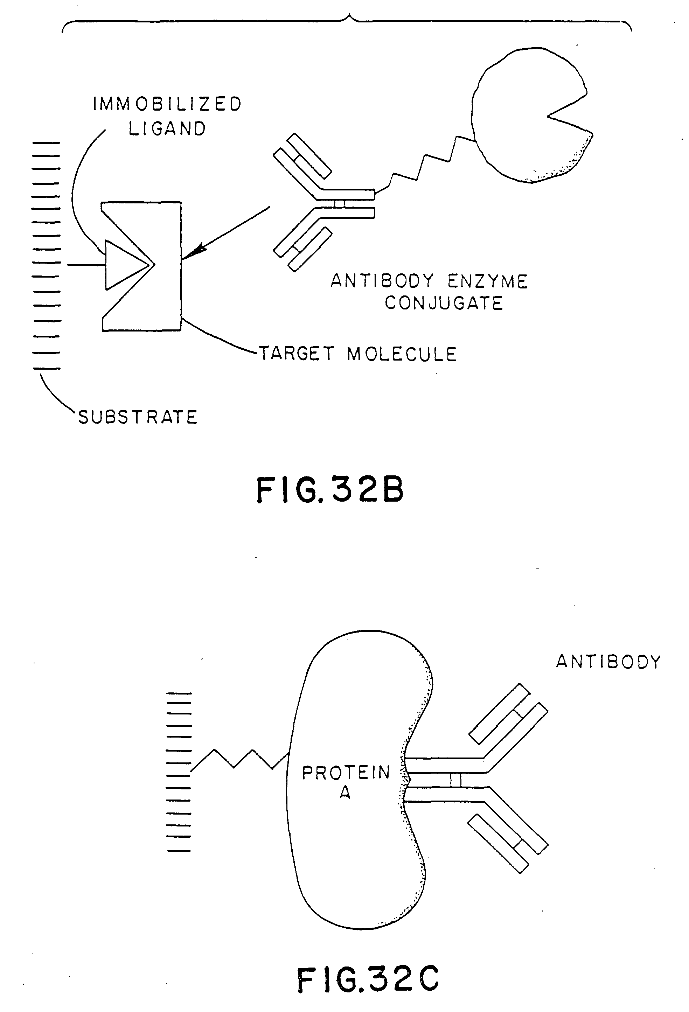

Turning now to Figures 32 through 35, still another embodiment of the apparatus for use in infusing beneficial agents into a patient is there illustrated. The device of this form of the invention is similar in many respects to the device shown in Figures 11 through 16 and like numbers are used to identify like components. As before selected additives are removably affixed to various forms of additive presentation means such as substrates, matrices and scaffolds which can be placed within the path of the fluid flowing through the device so that upon contact with the fluid, the additives are released at a controlled rate to the fluid.

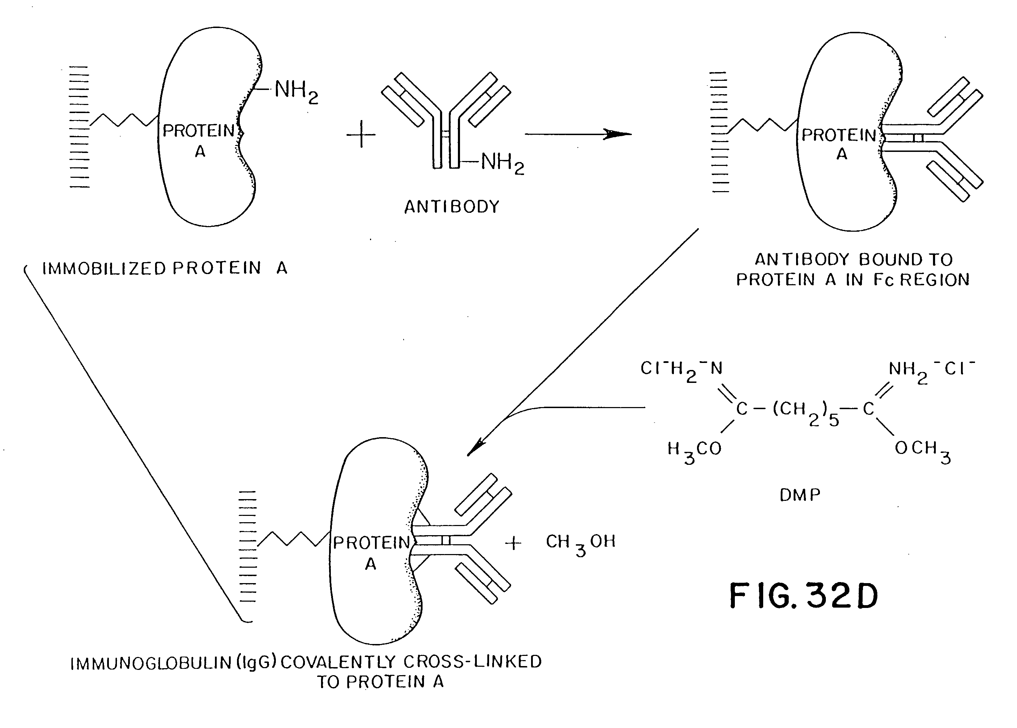

As best seen by referring to Figure 32, the apparatus comprises a base made of a first, generally planar member 700 and a second companion member 701 which includes a pair of longitudinally extending flow conduits or channels 702 and 704 which communicate with a transversely extending fluid transfer manifold conduit 706 (Figure 33). Conduit 706 is, in turn, connected with a fluid dispensing means shown here as including a fluid dispensing port 710.

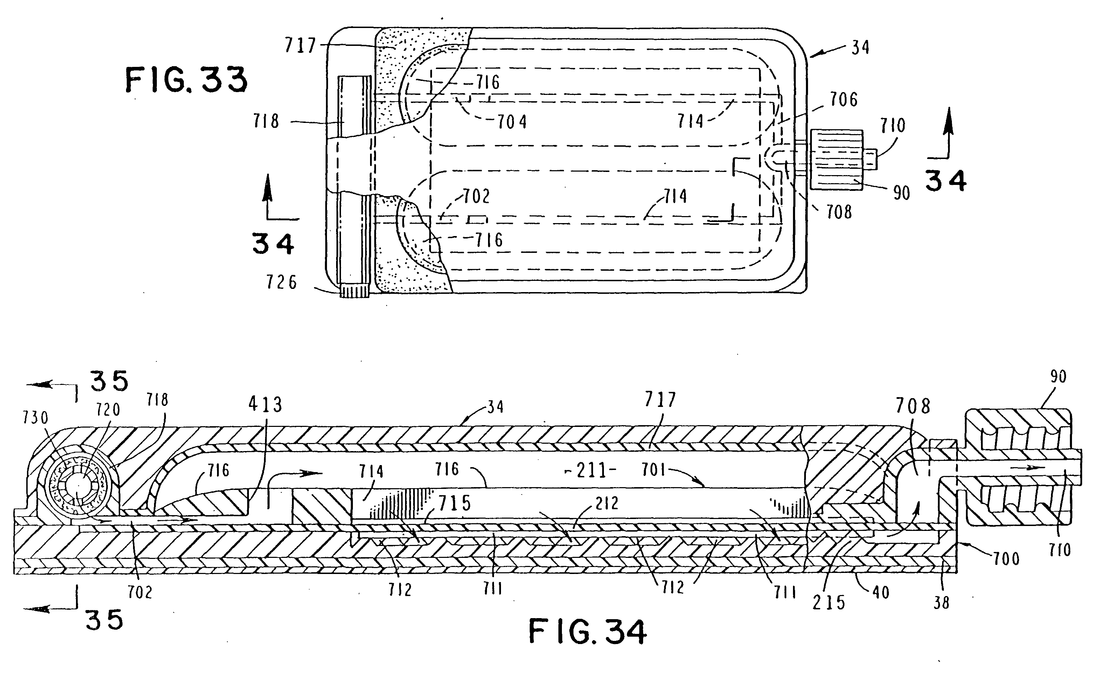

First base member 700 is also provided with a pair of longitudinally extending manifolds 711 which communicate with micro-channels 712 (Figure 34). Manifolds 711 and micro-channels 712 communicate with conduits 702 and 704 via flow control means of the general character described in connection with the embodiment of the invention shown in Figures 1 through 2. More particularly, the flow control means is here provided as a thin, multilayered or gradiated assembly 215 (see Figure 2) which is superimposed over manifolds 711 and micro-channels 712 in the manner shown in Figure 32. Assembly 212 is of the same construction and operates in the same manner as previously described. When the device is in a fluid discharge mode, fluid which is contained within chamber 211 (Figure 34) such as an elution buffer, diluent, or solvent containing the additive (the beneficial agent) is initially forced by the stored energy means through channels 714 in a pair of upstanding protuberances 716 formed on base member 701. As the liquid passes through channels 714, it enters a pair of longitudinally extending manifolds 715 which are formed in base member 701 and which align with manifolds 711 formed in base member 700.

The distendable membrane engagement means, shown here as protuberances 716 (Figure 32) perform the same function as previously described. It is to be understood that in some applications only a single protuberance is provided and in other applications no protuberance at all is required.

The stored energy means of this embodiment is of the same general character previously described and comprises an elastomeric distendable membrane 717. The membrane or membrane assembly is provided in the same manner as previously described herein.

As indicated in Figure 32, superimposed over the base and the stored energy source is a structural cover 34 of the character previously described having appropriate medicant and use labels 36. Affixed to the bottom of the base member 700 is a cushioning means or pad 38 having adhesive on both sides. A peel strip 40 is connected to the lower surface of pad 38. For certain applications, a thin protective film may be affixed over cover 34 to prevent ingress of liquids or other contaminants into the device.

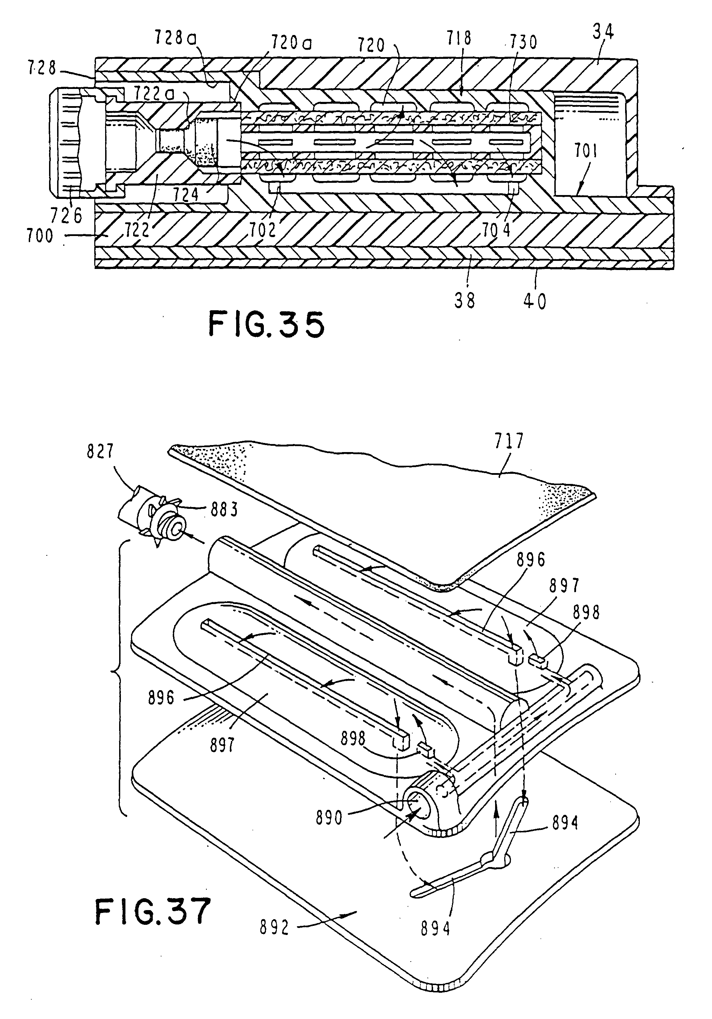

Like the previously described embodiments of the invention, the present embodiment includes filling means which enables chambers 211 to be filled with a selected parenteral liquid such as a buffer, a detergent, a solvent, a diluent or other eluting agents. To accomplish filling of the chamber, base member 701 includes an upstanding transversely extending portion 718 having a fluid passageway 720 extending therethrough. In this embodiment of the invention, the open end 720a of passageway 720 is closed by a novel filling-check valve means for controlling the flow of fluid into passageway 720, which here comprises a check valve housing 722, a check valve 724 and a safety luer cap 726 for interconnection with a standard luer fitting. As best seen in Figure 35, the fiilinq-check valve means is receivable within an enlarged diameter portion 728a of a structural support 728 which is closely receivable within portion 718.

As best seen by referring to Figure 34, passageway 720 is in communication with longitudinally extending channels 702 and 704. As before, channel 702 and 704 are, in turn, in communication with chambers 211. With this construction, an appropriate injectable such as a diluent or parenteral fluid or other elution agent, as indicated by the flow arrows, can be introduced into chambers 211 via luer cap 726 and passageway 720. As the parenteral fluid enters the check valve housing 722, check valve 724 will be moved away from seat 722a (to the right as viewed in Figure 35) to permit fluid flow inwardly of passageway 720. When the chambers are filled, the buildup of back pressure will cause the check valve to return to the closed position shown in Figure 35.

The adding means of the invention can take several different forms as, for example, the cylindrically shaped, functional support structure 730 which is inserted into passageway 720 and to which various additives, including beneficial agents such as drugs, biologically active materials, and chemical elements and compounds can be releasably connected. The adding means can also be of the character shown in Figure 19 wherein the additive is encapsulated within a sealed container.

In the form of the invention shown in Figure 32, the liquid medium flows around, about and through the substrate support. In the form of the invention shown in Figure 19, the liquid medium can be sealably contained within the container 630 and may include matrices suitable for affinity-based separation such as a synthetic support, a bead matrix material together with a suitable storage buffer or a gel. The details of these matrices will be described more fully hereinafter.

The additives can take various forms and, as previously mentioned, can be removably affixed to the functional support means in various ways to enable the use of separation techniques broadly defined by the term chromotography. Chromotography as used herein refers to a of separation techniques which are characterized by a distribution of the molecules to be separated between two phases, one stationary and the other mobile. Affinity chromotography involves the use of biological interactions and contemplates the use of affinity chromotography supports through which the eluting fluid flow. In the present embodiment of the invention, the additive presentation means assumes the character of an affinity chromotography support to which various ligands are attached. In the practice of affinity chromotography techniques, one of the members of the pair in the interaction, the ligand, is immobilized on a solid phase, while the other, the counterligand (most often a protein), is absorbed from the extract that is passing the substrate during the manufacturing process. Importantly, affinity chromotography techniques can include the use of highly versatile azlactone functional compounds, such as azlactone functional beads, a well as the use of a wide variety of other media for activation and coupling chemistry. Examples of ligands that can be attached to the affinity supports include antibodies, enzymes, lectins, nucleic acids, hormones and vitamins. Examples of important counterligands include antigens, virus, cells, cell surface receptors and the like. Chromotography and affinity chromotography techniques are described in detail in Protein Purification by Janson and Ryden, Copyright 1989 and reference should be made to this work to provide a working understanding of the techniques.

Polymeric azlactones are well known in the prior art. Their use in the production of homopolymers and copolymers has been described in a number of patents. See for example, U.S. Patent No. 3,488,327 (issued Jan. 6, 1970 to F. Kollinsky et al.); U.S. Patent No. 3,583,950 (issued January 8, 1971 to F. Kollinsky et al.); U.S. Patent No. 4,304,705 (issued December 8, 1981 to S.M. Heilmann et al.); U.S. Patent No. 4,737,560 (issued April 12, 1988 to S. M. Heilmann et al.); and U.S. Patent No. 5,013,795 issued May 7, 1991, Coleman, et al.

Azlactones, or oxazolones, are cyclic anhydrides of N-acylamino acids and have been used extensively in organic synthesis. The formation of a five-membered azlactone of particularly useful functionality for immobilization purposes can be accomplished through the reaction of a carboxylate group with a-methyl alanine using a two-step process (See Immobilized Affinity Ligand Techniques - Hermanson, Mallia and Smith, Copyright 1992.) One method of forming azlactone beads, the use of which has been previously mentioned herein, makes use of this process in the polymerization of monomers to first yield a carboxyl group on the matrix. In the second step, the azlactone ring is formed in anhydrous conditions through the use of a cyclization catalyst. Suitable cyclization agents that will drive this reaction include acetic anhydride, alkyl chloroformates, and carbondiimides. The process of forming these active groups and of making beaded polymeric supports containing them has been thoroughly described in patents assigned to 3M Corporation (U.S. Patent No. 4,871,824 and 4,737,560). These support materials are now available under the tradename "Emphase". U.S. Patent Nos. 5,045,615 and 5,013,795 which have been assigned to 3M Corporation also describe recent advances in this technology.

As pointed out in the 3M Corporation Patent No. 4,737,560, azlactone-functional polymer beads are useful reactive supports for the attachment of functional materials to provide novel adduct beads. The adduct beads are useful as complexing agents, catalysts, reagents, and as enzyme or other protein-bearing supports. The term "support" or "affinity support" as used in this sense is usually understood to refer to a combination of (1) a ligand (usually of some known molecular configuration), that is firmly attached (e.g., immobilized), often by covalent means, and (2) a matrix (usually a solid insoluble substance). Azlactone support matrix materials and coupling chemistry is also of special interest because of its accessible matrix surface area and effective ligand diversity that can be attached to that surface.

U.S. Patent No. 4,072,566 issued to Lynn on February 7, 1978, and entitled "Immobilized Biologically Active Proteins" discloses a method of bonding enzymes or other biologically active proteins to an inorganic support material using p-phenylenediamine. The support materials disclosed as useful in the invention include siliceous materials, stannic oxide, titania, manganese dioxide, and zirconia.

The functional support structure 730 of the present embodiment of the invention is uniquely constructed to permit the bonding thereto of enzymes or other biologically active proteins. This is accomplished by treating functional support 730 in the manner disclosed in the prior art patents identified in the preceding paragraphs with a compound having selected reactive functional groups such as azlactone functional compounds. In this way complexing agents, catalysts and biological materials such as enzymes, proteins, or other affinity absorbants, as well as biomacromolecules can be attached to the carrier for later removal and recovery.

When bonding certain biologically active proteins and other macro molecules, the use of spacer arms or leashes have been found to be very beneficial. Spacer arms or leashes are low-molecular-weight molecules that are used as intermediary linkers between a support material and an affinity ligand. Usually spacers consist of linear hydrocarbon chains with functionalities on both ends for each coupling to the support and ligand. First, one end of the spacer is attached chemically to the matrix using traditional immobilization chemistries; the other end is connected subsequently to the ligand using a secondary coupling procedure. The result is an immobilized ligand that sticks out from the matrix backbone by a distance equal to the length of the spacer arm chosen.

Referring to Figures 32A, 32B, 32C, and 32D the use of spacer arms to bond proteins and enzymes to the substrate is there schematically illustrated. The principal advantage in using a spacer arm is that it provides ligand accessibility to the binding site of a target molecule. When the target molecule is a protein with a biding site somewhat beneath its outer surface, a spacer is essential to extend the ligand out far enough from the matrix to allow interaction. As indicated in Figure 32A, when the ligand binding site S is buried or in a pocket 733 located just below the surface of the protein P, a ligand L that is either below the surface of the support material 735 (upper portion) or a ligand L-1 that is attached directly to the surface (middle portion) cannot reach the level of the binding site S on an approaching protein molecule. The result may be weakened interaction or no binding at all. Accordingly, in these instances, spacer arm 737 is required to provide the ligand L-2 accessibility to the binding site of the protein molecule (lower portion of Figure 32A). The details concerning use of spacer arms are fully set forth in Section 3.1.1 of the previously referred to work entitled Immobilized Affinity Ligand Techniques. This Section 3.1.1 is incorporated herein by reference.

Turning now to Figures 32A, 32B, 32C, and 32D, it is to be noted that immobilized Protein A can be used to immobilize an antibody molecule by taken advantage of the natural affinity of protein A for immunoglobulins. Incubation of a specific antibody with a protein A matrix will bind the antibody in the Fc region, away from the antigen biding sites. Subsequent cross-linking of this complex with DMP (dimethyl pimelimidate) yields a covalently attached antibody with the antigen binding sites facing outward and free to interact with antigen.

With rigid support materials, a spacer molecule may also provide greater flexibility, allowing the immobilized ligand to move into position to establish the correct binding orientation with a protein. The degrees of freedom that a hydrocarbon extender can provide are much greater than the movement possible within the polymeric backbone of a matrix.

The choice of spacer molecule can affect the relative hydrophilicity of the immediate environment of an immobilized ligand. Molecules containing long hydrocarbon chains may increase the potential for nonspecific hydrophobic interactions, especially when the affinity ligand is small and of low molecular weight. Selecting spacers that have more polar constituents, such as secondary amines, amide linkages, ether groups or hydroxyls will help keep hydrophobic effects to a minimum.

It is also important to consider the ionic effects a spacer molecule may impart to a gel. Spacers with terminal primary amine groups should be completely coupled with ligand or blocked by a nonrelevant molecule (e.g., acetic anhydride; see Section 3.1.1.9 of Immobilized Affinity Ligand Techniques) to eliminate the potential for creating a positive charge on the support. With small ligands, these residual charges can form a secondary environment that may cause considerable nonspecific interactions with proteins. The same holds true for spacers with terminal carboxylic groups. In general, a negatively charged spacer will cause less nonspecific protein binding than a positively charged one, but blocking excess remaining groups is still a good idea. A good blocking agent for use with carboxylic residues is ethanolamine, which laves a terminal hydroxyl group
(See Immobilized Affinity Ligand Techniques for a complete discussion of types of spacers and various immobilization and coupling protocols.)

Another work with fully describes alternate forms of protein immobilization is Protein Purification, Janeon and Ryden, Copyright 1989. As pointed out in this work at Page 310:
"Ligand-protein interaction is often based on a combination of electrostatic, hydrophobic and hydrogen bonds. Agents which weaken such interactions might be expected to function as effective non-specific eluants."

This work provides a further teaching of the techniques described herein.

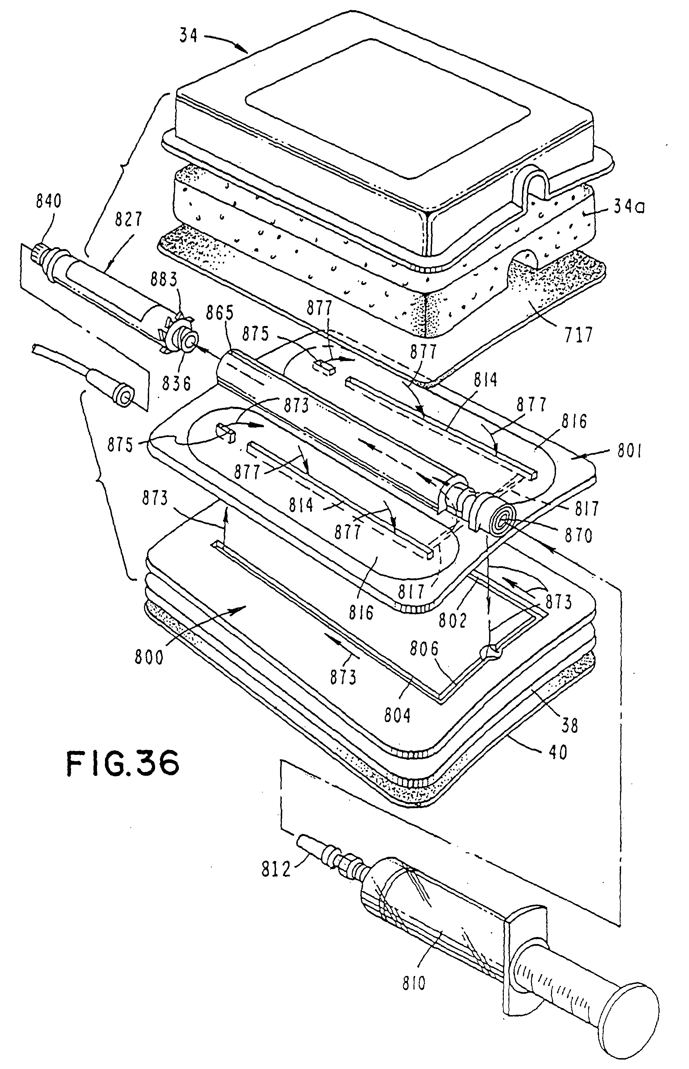

It is important to recognize that, as used in the present form of the invention, affinity supports are capable of total binding capacity of a magnitude that enables attachment to the support of additives in substantial amounts for subsequent release, recovery and infusion of such beneficial agents at a level which can be therapeutically efficatious. Turning now to Figure 36, yet a device not belonging to the invention which is similar in many respects to the embodiment in Figure 19 is shown. In this embodiment not belonging to the invention the distendable membrane is a single elastomeric sheet 717. However, as will become apparent, fluid flowing through the device is totally different. In this latest form of the invention, selected additives are removably affixed to various forms of support structures which are, in turn, contained within a novel additive subassembly that can be inserted into the outlet port of the device in a manner to place the additives within the path of the elution fluid flowing outwardly of the device from the fluid reservoirs. In this way the additives, such as beneficial agents or other pharmaceutically active materials can remain coupled with an appropriate matrix support and sealed in the prepackage additive subassembly, which has been precharged with an elution buffer, aqueous diluent, solvent or other parenteral liquid. In other words, unlike the previously described embodiments in which the additive is mixed with the fluid medium during the filling step, here the additive is mixed with the elution fluid as the fluid flows from the prefilled or precharged reservoirs of the device.

As best seen by referring to Figure 36, the apparatus comprises a base made up of a first, generally planar member 800 and a second companion member 801. Member 800 includes a pair of a longitudinally extending flow channels 802 and 804 which communicate with a transversely extending fluid transfer passageway 806. Passageway 806 is, in turn, connected with a fluid inlet 808 (Figure 40) which is in communication with a fluid charging or filling means shown here as a syringe assembly 810 which includes a blunt cannula 812.

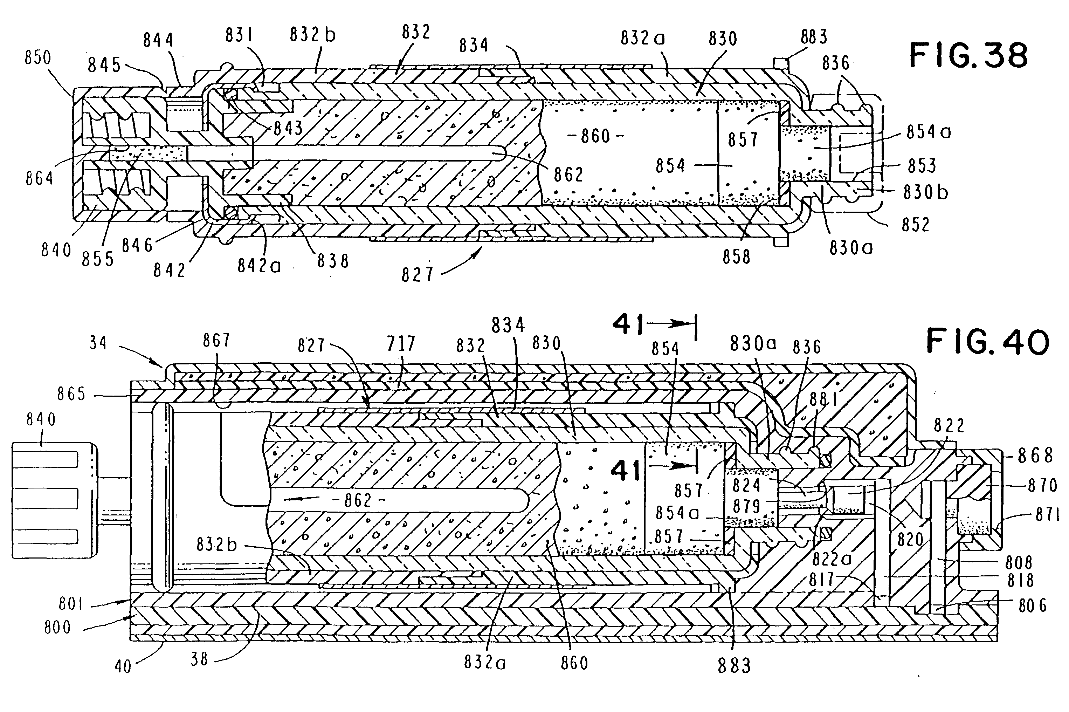

Reservoir outlet passageways for fluid flowing outwardly from the reservoir are provided in the form of longitudinally extending flow channels 814 provided in a pair of protuberances 816 which are integrally formed with base member 801. Channels 814 communicate with transversely extending channels 817 which, in turn, communicate with a central passageway 818 (Figure 40) which leads to a check valve housing 820. Reciprocally mounted within housing 820 is a check valve 822 which includes a stem portion 824. In a manner presently to be described check valve 822 controls fluid flow between the fluid reservoirs of the device and the interior of the additive subassembly shown in Figure 38 and generally designated in the numeral 827.

Additive subassembly 827 comprises a glass vial 830 which is closely received within a plastic shell 832 that is constructed in two parts 832a and 832b. Parts 832a and 832b are joined together by a gummed medicament label 834. The fluid inlet end of glass vial 830 is provided with external threads 836 while the outlet end of the vial is open to receive the skirt portion 838 of a luer fitting 840. Skirt portion 838 is maintained in position within the outlet end of vial 830 by an aluminum crimp seal 842, the circumferentially extending edge 842a thereof being adapted to be crimped over into a circumferential groove 831 provided in glass vial 830. An O ring seal 843 surrounds skirt 838 and abuts the edge of the glass vial to prevent leakage between the vial and the luer fitting. Structure 850 also sealably closes the lumen 864.

The outlet portion 832b of shell 832 is provided with a reduced diameter portion 844 that defines an internal shoulder 846 that abuts crimp seal 842. Reduced diameter portion 844 is provided with a circumferentially extending score line 845 that permits the outer, cap-like end 850 of portion 844 to be removed at the time of use of the additive subassembly 827 to expose luer fitting 840. A tear-off cap 852 is also provided at the inlet end of the additive subassembly. When this cap is removed, the threads 836 of vial 830 are exposed. Cap 852 also functions to sealably close inlet passageway 853.

Closely received within the inlet end of vial 830 is resistance and flow rate control means here provided as a porous plug 854 having a reduced diameter portion 854a which is closely receivable within neck 830a of vial 830. Plug 854 can be constructed from any suitable porous material such as ceramic, glass, PTFE, carbon or other inert material and functions to controllable resist and precisely regulate fluid flow from the fluid reservoirs of the device toward the additive support or functional substrate. An elastomeric ring seal 857 is interposed between plug 854 and a shoulder 858 defined interiorly of vial 830 to prevent leakage between the plug and the shoulder.

The additive support or matrix, here identified by the numeral 860, is closely received within vial 830 and abuts plug 854 in the manner shown in Figure 40. As before, support 860 can be of various constructions as previously described, including azlactone beads and distal porous glass frit containment means. Support 860 removably carries selected additives, such as the beneficial agents previously described herein. A centrally disposed fluid flow channel 862 is provided in support 860 and is adapted to communicate with the fluid outlet passageway 864 of the dispensing means, or luer fitting assembly 840.

The distendable membrane engagement means or protuberances 816 perform the same function as previously described as does the unique stored energy means. The stored energy means, or elastomeric member 717, of this embodiment is of the general character previously described in connection with the embodiment of Figure 1 and cooperates with base member 801 to define the fluid chambers or reservoirs of the device. The stored energy means can also be of a laminate construction of the character previously described herein.

As indicated in Figure 36, superimposed over the base and stored energy source 717 is a structural cover 34 of the character previously described. Affixed to the bottom of base member 800 is a cushioning means or pad 38 having adhesive on both sides. A peel strip 40 is connected to the lower surface of pad 38.

Turning now to Figures 36 and 40, base member 801 includes an upstanding, longitudinally extending portion 865 having a generally cylindrical chamber 867 extending therethrough. In this embodiment of the invention, chamber 867 is uniquely designed to closely receive the additive subassembly 827 that sealably contains the additive to be added to the elution fluid flowing from the fluid reservoirs outwardly of the device. At the inlet end of chamber 867 there is provided a septum housing 868 adapted to support an elastomeric septum 870 which is accessible by cannula 812 of the filling syringe via an opening 871. When cannula 812 pierces septum 870, fluid will be permitted to flow from the syringe into passageway 808 and thence to the fluid reservoirs in the manner indicated by the arrows 873 of Figure 36 via channels 802, 804 and inlet ports 875. As the fluid flows through ports 875, distendable membrane 717 will be urged into its distended configuration in the manner previously discussed.

As the reservoirs are charged, fluid will flow downwardly though channels 814 in protuberances 816 and into passageway 818 as indicated by the arrows 877 of Figure 36. This fluid under pressure will urge check valve 822 into its closed position with shoulders 822a in sealing engagement with seats 879 (Figure 42). With the check valve 822 in the closed position, fluid cannot flow toward cylindrically shaped chamber 867 of base member 801.

In using the apparatus of the embodiment of the invention shown in Figures 36, 38, 39, 41 and 42, sealing caps 850 and 852 are removed to expose the luer fitting 840 and the threaded end of glass vial 830b of the additive subassembly 827. The additive subassembly 827 is then inserted into chamber 867 so that threads 836 engage mating threads 881 of portion 865. Rotation of the vial will then cause stem 824 of check valve 822 to move the check valve from the closed position shown in Figure 42 to the open position shown in Figure 40. As the vial is rotated relative to portion 865 of base 801, locking tabs 883 provided on the vial subassembly (Figure 38) engage resiliently deformable tabs 885 provided internally of chamber 867 (see Figure 41). Tabs 885 are designed to permit rotation of the vial subassembly in one direction but to block rotation in the opposite direction. With this construction, once the vial assembly has been interconnected with the base assembly, it cannot be removed.

With the vial assembly 827 locked in place within chamber 867, and the check valve open, the sterile diluent is urged from the reservoirs by the distendable membrane 717 and introduced into check valve housing 820 via channels 814, 817 and 818. The diluent then flows controllably through the porous plug 854 and longitudinally of the additive presentation means, or substrate 860 where it efficiently intermixes with the additive carried by the substrate. The mixture thus formed flows through passageway 862 into passageway 864 of the luer fitting and outwardly of the device.

Forming an important part of this embodiment of the invention is the unique feature for commonality of use with selected assemblies that contain both flow rate control means and an additive having an extended release rate. By appropriate selection of this assembly, this feature allows for individual control of the rate of dosing of the beneficial agent into the elution diluent independent of the rate of flow of the elution diluent.

The manual facility to individually control, through proper assembly selection, the delivery of both drug dosing rate and diluent dispensing rate, over a given time period, is a desireable feature. In practice, alternative vial cartridges (827 - Figure 3-6) can be provided which control fluid flow rate at a given level and also additive release rates at a desired predetermined level. This capacity can insure delivery of a required dosage within a therapeutically acceptable time period over a broad range of fluid flow rates.

Additionally, the control of dosage rates can also help prevent over-concentration of an administered drug which can result in patient local or systemic toxicity.

Selection of an appropriate vial containing the predetermined extended release over-time type additives combined with an appropriate preselected flow rate control format over a wide range of delivery protocols can facilitate the expanded ambulatory delivery opportunities of various therapeutic agents. New delivery protocols can also be established for pharmaceutical agent delivery modes and administration regimes.

The extended release additives can comprise bounded proteins or other absorbants which can be attached to and eluded from the matrix over time in a variety of ways well known to those skilled in the art.

The rate control means is here shown at both the proximal end of the vial (854), and at the distal end of the vial (855). However, the rate control means can be located proximate either end of the vial for selected rate control, filtering and elutent residence time in the vial.

Referring to Figure 37 another form of the invention, similar to that just described is shown. This device operates in basically the same way as the device of Figure 36 save that filling is accomplished through a side inlet port 890 rather than an end inlet port of the character shown in Figure 36. The vial subassembly 827 is identical to that previously described and fluid is urged from the reservoirs by distendable membrane 717 and flows outwardly of the device through the vial subassembly. The base member 892 of the device is of slightly different construction, having angularly inclined flow channels 894 which receive the diluent flowing from the reservoirs through channels 896 formed in protuberances 897. Filling of the reservoirs is accomplished by a filling means which urges fluid flow into port 890 and into the reservoirs via inlet ports 898. The arrows of Figure 37 clearly depict the direction of fluid flow into and out of the device.

Referring to figures 43 through 49, another form of the device of the-invention is there shown. This device is very similar to that shown in Figures 32 through 35 save that filling is accomplished by using a filling means which comprises a novel needle assembly, rather than by using the luer connector assembly shown in Figure 32.

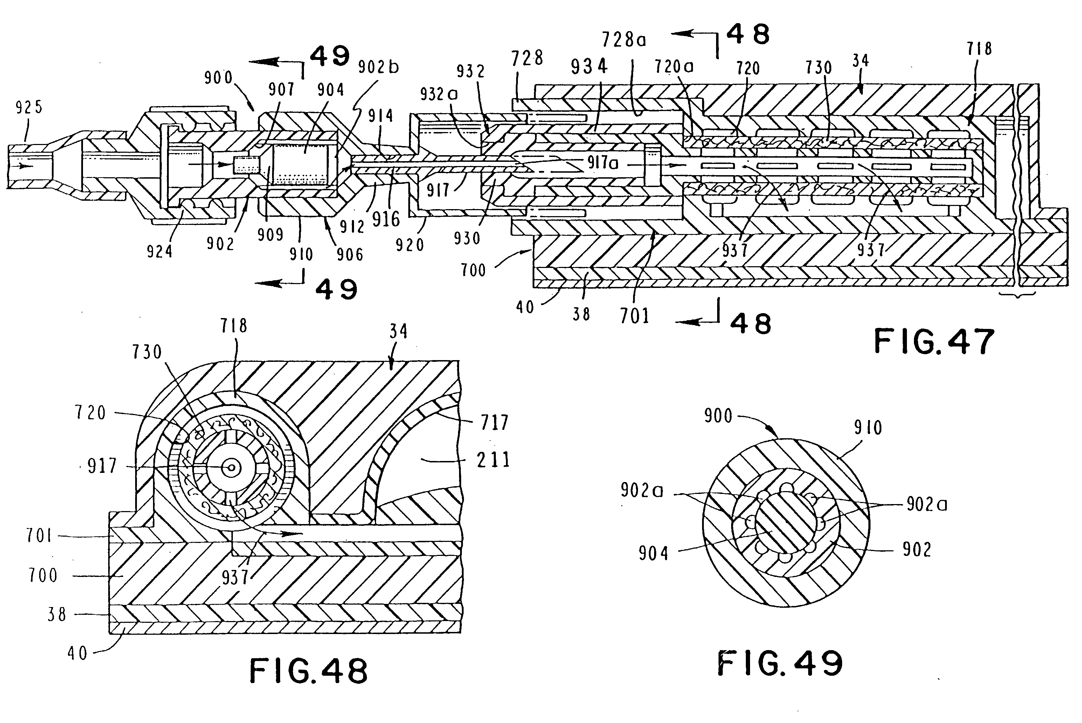

As best seen in Figure 47, the luer cap an internal check valve assembly of the device of Figure 32 has been replaced by a septum assembly which is mounted within the inlet passageway 720a of the device. Due to the close similarity between the devices, like numerals have been used in Figures 43 through 49 to identify like components shown in Figures 32 through 35.

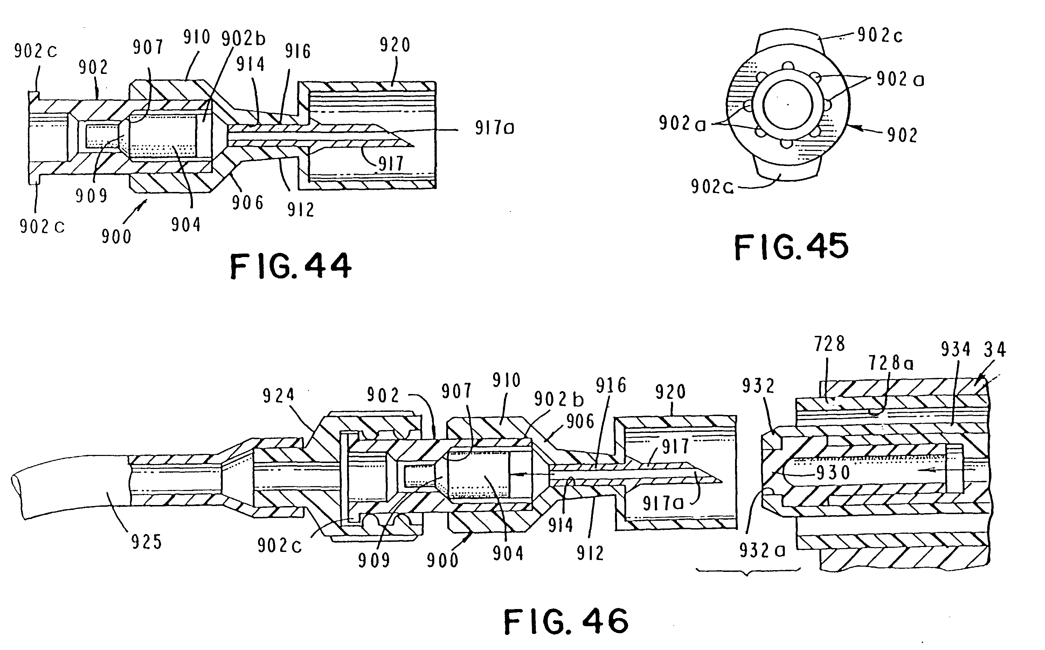

The injection assembly, here designated by the numeral 900, is best seen in Figure 44 and includes a check valve housing 902, a check valve 904 reciprocally movable within housing 902 and a needle assembly 906 connected to housing 902. Housing 902 includes an internal seat 907 against which a shoulder 909 of the check valve seats when the valve is in the closed position shown in Figure 46. By referring to Figure 45, which is an end view of housing 902, it can be seen that a plurality of circumferentially-spaced flow channels 902a are provided in the wall of the housing. (See also Figure 49).

Needle assembly 906 comprises a collar 910 which is receivable over one end of housing 902 and is connected thereto by any suitable means such as adhesive bonding. Integrally formed with collar 910 is a neck portion 912 having a bore 914 adapted to receive the inboard end 916 of a hollow needle 917. Integral with neck portion 912 is a safety needle shroud 920 which surrounds needle 917. Shroud 920 is specifically configured to eliminate accidental needle sticks. Also shroud 920 is adapted to fit over port structure 934 in the manner shown in Figure 47 where the needle 917 has punctured septum 930. The internal passageway 917a of the needle communicates with the internal chamber 920b of the check valve housing to permit fluid flow through the needle to the fluid reservoirs of the device when the check valve is in an open position.

Turning to Figures 43 and 46, it is to be observed that check valve housing 902 includes circumferentially spaced luer lugs 902c which permit interconnection of a coupler member 924 which the needle assembly. Member 924 is, in turn, connected with a length of transfer cannula tubing 925 that connects to a source of diluent or other parenteral liquid.

In using the apparatus of this last form of the invention, the protracted needle is caused to penetrate the septum 930 of the septum assembly in the manner shown in Figure 47. The septum 930 is of conventional construction and is maintained in position within the open end 720a of passageway 720 by a septum housing 932 having a skirt portion 934 which is receivable within enlarged diameter portion 728a of structural support 728 which is, in turn, closely receivable within portion 718 of the base assembly. Septum housing 932 has an inlet opening 932a which permits needle 917 to penetrate the septum.

As indicated in Figures 43 and 48, superimposed over the base and stored energy source 717 is a structural cover 34 of the character previously described. Affixed to the bottom of base member 700 is a cushioning means or pad 38 having adhesive on both sides. A peel strip 40 is connected to the lower surface of pad 38.

With the construction shown in the drawings, an appropriate injectable such as diluent, parenteral fluid, or other elution fluid can be introduced into chambers 211 using the needle assembly 900. As the fluid flowing from the fluid source enters the check valve housing 902 of the needle assembly, check valve 904 will be moved away from seat 907 to the open position as shown in Figure 47 to permit fluid flow inwardly of the hollow needle. When the chambers are filled, the buildup of system back pressure will cause the check valve to return to the closed position shown in Figure 46 and the needle can be withdrawn from the septum.

Referring to Figures 47 and 48, the adding means of the invention can take several different forms as, for example, the cylindrically shaped, functional support structure 730 which is inserted into passageway 720 and to which various additives, including beneficial agents such as drugs, biologically active materials, and chemical elements and compounds can be releasably connected. The additives can be of the character described in connection with the previously described embodiments of the invention including those shown in Figures 32 and 35 and can be removably affixed to support structures of various configurations including synthetic porous matrices such as structure 730 which can be placed within the path of the fluid flowing through the device. As the fluid flows toward the reservoirs in the manner indicated by the arrows 937 of Figures 47 and 48, the additives are released at a controlled rate over time to the fluid. This can be accomplished by slow diffusion mass transport, slow equilibration kinetics or other types of absorbent disassociation techniques.

Having now described the invention in detail in accordance with the requirements of the patent statutes, those skilled in this art will have no difficulty in making changes and modifications in the individual parts of their relative assembly in order to meet specific requirements or conditions. Such changes and modifications may be made without departing from the scope of the invention, as set forth in the claims.

## Claims

1. A device for use in controllably infusing medicinal fluids into a patient comprising:
(a) a base (300) having a width
(b) and dispensing means operably associated with a fluid outlet (310) for dispensing fluid from the device,
(c) energy storage means for forming, in conjunction with said base, a low profile fluid chamber (211) which includes a fluid outlet (213),
**characterized in that**
(d) said energy storage means comprises a multi-layer construction (318) having at least two distinct distendable membranes (320, 322) superimposed over said base (300), said distendable membranes being distendable by the fluids to be infused, thereby establishing internal stresses which tend to return said members to a less distended configuration, said distendable membranes acting to controllably urge fluid from said chamber through said outlet.

2. A device as defined in claim 1 further including a flow control means is disposed internally of said chamber for controlling a rate of flow of fluid out of said device, said internal flow control means comprising at least one thin permeable membrane superimposed over said base.

3. A device as defined in claims 1 or 2 further including an external flow control means disposed intermediate said fluid outlet and said dispensing means for controlling a rate of flow of fluid out of said device.

4. A device as defined in one of claims 1-3 in which at least one of said planar-shaped, distendable membranes is permeable to gas.

5. A device as defined in claim 4 in which at least one of said distendable membranes has a first permeability constant.

6. A device as defined in claim 5 in which at least one of said distendable membranes has a second permeability constant.

7. A device as defined in claim 1 wherein said device is used for infusing fluids into an ambulatory patient at a controlled rate wherein
(a) said base has said fluid inlet and said fluid outlet (310) interconnected by a fluid flow path;
(b) said distendable membrane (320, 322) is constructed of an elastic material which is fitted over said base (300) to define said chamber and is in communication with said fluid inlet and said fluid outlet (310), and wherein said device further includes:
(c) filling means for introducing fluid into said fluid inlet; and
(d) adding means disposed within said fluid flow path for adding an additive to fluid flowing therethrough.

8. A device as defined in claim 7 in which said adding means comprises an additive and an additive presentation means for presenting said additive to the fluid, said additive presentation means being disposed within said fluid flow path so that at least a part of said additive will be added to said fluid introduced into said fluid inlet.

9. A device as defined in claim 7 in which said adding means comprises an additive and an additive presentation means for presenting said additive to the fluid, said additive presentation means having exposed surfaces, said additive being removably connected to said exposed surfaces.

10. A device as defined in claim 7 in which said adding means comprises a structural support having exposed surfaces and a beneficial agent present on said exposed surfaces, said structural support being disposed within said fluid flow path so that at least a portion of said beneficial will be added to said fluid introduced into said fluid inlet.

11. A device as defined in claim 7 in which said adding means comprises a polymer support and an additive carried by said polymer support, said polymer support being disposed within said fluid flow path so that at least a portion of said additive will be added to the fluid flowing through said fluid flow path.

12. A device as defined in claim 7 in which said adding means comprises a member having exposed surfaces and a biologically active material present on said exposed surfaces, said member being disposed within said fluid flow path so that said biologically active material will be added to said fluid introduced into said fluid inlet.

13. A device as defined in claim 7 wherein said device is used for use in infusing fluids into a patient at a controlled rate, further comprising:
a fluid passageway (420) interconnecting said fluid inlet and said fluid outlet (310), whereby said chamber is in communication with said fluid passageway (420) and fluid is introduced into said chamber under pressure through said fluid passageway (420); and wherein
said adding means is mounted within said base, said adding means comprising:
additive presentation means disposed within said fluid passageway for presenting an additive to the fluid flowing therethrough; and
an additive removably interconnected to said additive presentation means, said additive comprising a beneficial agent.

14. A device as defined in claim 11 in which said distendable membrane includes a surface in contact with the fluid introduced by said filling means and in which said additive is removably interconnected with said surface.

15. A device as defined in claim 13 in which said additive presentation means comprises a flow rate control membrane disposed between said fluid outlet of said base and said chamber, said flow rate control membrane having exposed surfaces, said additive being removably connected to said exposed surfaces.

16. A device as defined in claim 13 in which said additive presentation means comprises a surface in said base, said additive being removably connected to said surface.

17. A device as defined in claim 13 in which said adding means comprises a vial assembly having said additive presentation means sealably contained therein.

18. A device as defined in claim 17 in which said vial assembly comprises a glass vial having first and second ends sealed by first and second sealing members.

19. A device as defined in claim 18 in which said base includes a chamber for receiving said glass vial.

20. A device as defined in claim 19 in which a hollow needle is mounted within said chamber (211) for piercing said second sealing member, said hollow needle having a fluid passageway (520) in communication with said fluid passageway of said base.

21. A device as defined in claim 7, wherein said device is disposed for infusing fluids into an ambulatory patient and wherein
said base (501) has an upstanding portion (519) including a fluid inlet passageway (520), said fluid outlet and said fluid inlet passageway (520) being interconnected by said fluid flow path
and said filling means includes a filling check valve means for controlling the flow of fluid into said fluid inlet passageway.

22. A device as defined in claim 19 in which said adding means comprises an additive and an additive presentation means for presenting said additive to the fluid, said additive presentation means being disposed within said fluid inlet passageway (520) intermediate said fluid outlet and said filling check valve means.

23. A device as defined in claim 19 in which said adding means comprises an additive and an additive presentation means for presenting said additive to the fluid, said additive being removable from said additive presentation means using affinity chromographic techniques.

24. A device as defined in claim 23 in which said additive is removably connected to said additive presentation means using aziactone functional compounds.

25. A device as defined in claim 23 in which a ligand is connected to said additive presentation means and a target molecule is connected to said ligand.

26. A device as defined in claim 25 in which a spacer is connected to said support and in which enzymes are connected to said spacer.

27. A device as defined in claim 25 in which a spacer is connected to said support and in which a protein is connected to said spacer.

28. A device as defined in claim 27 in which an antibody is bound to said protein.

29. A device as defined in claim 7 wherein said device is used for infusing fluids into an ambulatory patient at a controlled rate and wherein
said fluid outlet (608) is a fluid outlet passageway and a fluid passageway interconnecting said fluid inlet and said fluid outlet passageway, whereby said chamber (620) is in communication with said fluid passageway and wherein
said adding means is disposed within said fluid outlet passageway for adding an additive to fluid flowing therethrough, said adding means comprising a support and an additive removably connected to said support.

30. A device as defined in claim 29 in which said filling means comprises a syringe assembly.

31. A device as defined in claim 29 in which an upstanding portion (626) receiving said adding means extending transversely of the base member (601) of the device.

32. A device as defined in claim 29 in which said upstanding portion (626) extending longitudinally of said base member (601).

## Patentansprüche

1. Vorrichtung für den Einsatz zur steuerbaren Infusion medizinischer Lösungen in einen Patienten, welche umfasst:
(a) eine Grundplatte (300) mit einer gewissen Größe
(b) und Ausströmmittel, die bedienbar mit einer Flüssigkeitsaustrittsöffnung (310) zum Ausfließen der Flüssigkeit aus der Vorrichtung verbunden sind,
(c) Energiespeichermittel, um in Verbindung mit der besagten Grundplatte eine flache Strömungskammer (211) zu bilden, welche eine Flüssigkeitsaustrittsöffnung (213) enthält,
**dadurch gekennzeichnet, dass**
(d) das besagte Energiespeichermittel einen Mehrschichtenaufbau (318) aufweist, der mindestens zwei unterschiedliche dehnbare Membranen (320, 322) enthält, die der besagten Grundplatte (300) überlagert sind, wobei die besagten dehnbaren Membranen durch die zu infundierenden Flüssigkeiten gedehnt werden können, wodurch innere Spannungen aufgebaut werden, die die Neigung haben, die besagten Teile in eine weniger gedehnte Konfiguration zu bringen, wobei die dehnbaren Membranen dahingehend wirken, dass sie die Flüssigkeit in steuerbarer Weise durch die besagte Austrittsöffnung hindurch aus der besagten Kammer heraus drücken.

2. Vorrichtung nach Anspruch 1, die außerdem ein Mittel zur Strömungssteuerung enthält, das im Innern der besagten Kammer zur Steuerung des Volumenstroms der aus der besagten Vorrichtung austretenden Flüssigkeit steuert, wobei das besagte innere Mittel zur Strömungssteuerung zumindest eine dünne permeable Membran enthält, die über der besagten Grundplatte liegt.

3. Vorrichtung nach Anspruch 1 oder 2, die außerdem ein äußeres Mittel zur Strömungssteuerung enthält, das zwischen der besagten Flüssigkeitsaustrittsöffnung und dem besagten Ausströmmittel für die Steuerung des Volumenstroms der aus der besagten Vorrichtung austretenden Flüssigkeit angeordnet ist.

4. Vorrichtung nach einem der Ansprüche 1 bis 3, in der wenigstens eine der planar gestalteten dehnbaren Membranen für Gas durchlässig ist.

5. Vorrichtung nach Anspruch 4, bei der mindestens eine der dehnbaren Membranen eine erste Permeabilitätskonstante hat.

6. Vorrichtung nach Anspruch 5, bei der mindestens eine der dehnbaren Membranen eine zweite Permeabilitätskonstante hat.

7. Vorrichtung nach Anspruch 1, bei der die besagte Vorrichtung benutzt wird, um in einen ambulant versorgten Patienten Flüssigkeiten mit einem gesteuerten Volumenstrom zu infundieren, wobei
(a) die besagte Grundplatte die besagte Flüssigkeitseintrittsöffnung und die besagte Flüssigkeitsaustrittsöffnung (310) aufweist, die untereinander durch einen Flüssigkeitsströmungspfad verbunden sind;
(b) die besagte dehnbare Membran (320, 322) aus einem elastischen Material aufgebaut ist, das über der besagten Grundplatte (300) befestigt ist, um die besagte Kammer festzulegen, und mit der besagten Flüssigkeitseintrittsöffnung und der besagten Flüssigkeitsaustrittsöffnung (310) in Verbindung steht, und wobei die besagte Vorrichtung außerdem enthält:
(c) Einfüllmittel zum Einführen der Flüssigkeit in die besagte Flüssigkeitseintrittsöffnung; und
(d) Dosiermittel, die innerhalb des besagten Flüssigkeitsströmungspfades für das Zufügen eines Zusatzstoffes zu der hindurchströmenden Flüssigkeit angeordnet sind.

8. Vorrichtung nach Anspruch 7, bei der die besagten Dosiermittel einen Zusatzstoff und ein Darstellungsmittel zum Darstellen des besagten Zusatzstoffes in der Flüssigkeit enthält, wobei das besagte Darstellungsmittel für den Zusatzstoff innerhalb des besagten Flüssigkeitsströmungspfades so angeordnet ist, dass mindestens ein Teil des besagten Zusatzstoffes zu der in die besagte Flüssigkeitseintrittsöffnung eingefüllten Flüssigkeit zugegeben wird.

9. Vorrichtung nach Anspruch 7, bei der die besagten Dosiermittel einen Zusatzstoff und ein Darstellungsmittel für Zusatzstoffe zum Darstellen des besagten Zusatzstoffes zu der Flüssigkeit enthält, wobei die besagten Darstellungsmittel für Zusatzstoffe exponierte Flächen haben und der besagte Zusatzstoff mit den besagten exponierten Flächen entfernbar verbunden ist.

10. Vorrichtung nach Anspruch 7, bei der die besagten Dosiermittel einen strukturellen Träger enthalten, welcher exponierte Flächen und eine begünstigende Substanz auf den exponierten Flächen aufweist, wobei der besagte strukturelle Träger innerhalb des besagten Flüssigkeitsströmungspfades so angeordnet ist, dass wenigstens ein Teil der besagten begünstigenden Substanz zu der besagten Flüssigkeit gegeben wird, die in die besagte Flüssigkeitseintrittsöffnung eingefüllten wird.

11. Vorrichtung nach Anspruch 7, bei der die besagten Dosiermittel einen Träger aus Polymer und einen Zusatzstoff enthalten, der von dem besagten Träger aus Polymer getragen wird, wobei der besagte Träger aus Polymer innerhalb des besagten Flüssigkeitsströmungspfades so angeordnet ist, dass wenigstens ein Teil des besagten Zusatzstoffes der besagten Flüssigkeit zugegeben wird, die durch den besagten Flüssigkeitsströmungspfad strömt.

12. Vorrichtung nach Anspruch 7, bei der die besagten Dosiermittel ein Teil enthalten, das exponierte Flächen und ein biologisch aktives Material auf den besagten exponierten Flächen aufweist, wobei das besagte Teil innerhalb des besagten Flüssigkeitsströmungspfades so angeordnet ist, dass das biologisch aktive Material zu der besagten Flüssigkeit gegeben wird, die in die besagte Flüssigkeitseintrittsöffnung eingefüllt wird.

13. Vorrichtung nach Anspruch 7, bei der die besagte Vorrichtung dazu benutzt wird, in einen Patienten Flüssigkeiten mit einem gesteuerten Volumenstrom zu infundieren, die ferner enthält: einen Flüssigkeitsdurchgang (420), der die besagte Flüssigkeitseintrittsöffnung mit der besagten Flüssigkeitsaustrittsöffnung (310) verbindet, wodurch die besagte Kammer mit dem besagten Flüssigkeitsdurchgang (420) in Verbindung steht und unter Druck Flüssigkeit in die besagte Kammer durch den besagten Flüssigkeitsdurchgang (420) eingeführt wird und bei der die besagten Dosiermittel innerhalb der besagten Grundplatte montiert sind, wobei die besagten Dosiermittel enthalten:
Mittel zur Darstellung des Zusatzstoffes, die innerhalb des besagten Flüssigkeitsdurchgangs angeordnet sind, um einen Zusatzstoff zu der hindurchströmenden Flüssigkeit darzustellen; und
einen Zusatzstoff, der mit dem besagten Darstellungsmittel für Zusatzstoffe entfernbar verbunden ist, wobei der besagte Zusatzstoff eine begünstigende Substanz enthält.

14. Vorrichtung nach Anspruch 11, bei der die besagte dehnbare Membran eine Fläche enthält, die sich mit der Flüssigkeit in Kontakt befindet, die durch das besagte Einfüllmittel eingeführt wird, und bei der der besagte Zusatzstoff mit der besagten entfernbar Fläche verbunden ist.

15. Vorrichtung nach Anspruch 13, bei der das besagte Darstellungsmittel für Zusatzstoffe eine Steuermembran für den Volumenstrom enthält, die zwischen der besagten Flüssigkeitsaustrittsöffnung der besagten Grundplatte und der besagten Kammer angeordnet ist, wobei die besagte Steuermembran für den Volumenstrom exponierte Flächen hat und der besagte Zusatzstoff mit den besagten exponierten Flächen entfernbar verbunden ist.

16. Vorrichtung nach Anspruch 13, bei der das besagte Darstellungsmittel für Zusatzstoffe eine Fläche in der besagten Grundplatte enthalten, wobei der besagte Zusatzstoff mit der besagten Fläche entfernbar verbunden ist.

17. Vorrichtung nach Anspruch 13, bei der die besagten Dosiermittel eine Glasfläschchenanordnung enthalten, in der sich die besagten Darstellungsmittel für Zusatzstoffe dicht verschließbar befinden.

18. Vorrichtung nach Anspruch 17, bei der die besagte Glasfläschchenanordnung ein Glasfläschchen enthält, welches ein erstes und ein zweites Ende hat, die mit einem ersten und einem zweiten Verschlussteil dicht verschlossen sind.

19. , Vorrichtung nach Anspruch 18, bei der die besagte Grundplatte eine Kammer für die Aufnahme des besagten Glasfläschchen enthält.

20. Vorrichtung nach Anspruch 19, bei der eine Hohinadel in der besagten Kammer (211) zum Durchstechen des besagten zweiten Verschlussteils eingebaut ist, wobei die besagte Hohlnadel einen Flüssigkeitsdurchgang (520) in Verbindung mit dem besagten Flüssigkeitsdurchgang der besagten Grundplatte hat.

21. Vorrichtung nach Anspruch 7, bei der die besagte Vorrichtung für die Vornahme der Infusion von Flüssigkeiten in einen ambulant versorgten Patienten angeordnet ist und in der die besagte Grundplatte (501) einen aufrechten Teil (519) hat, der einen Flüssigkeitseintrittsdurchgang (520) enthält, wobei die besagte Flüssigkeitsaustrittsöffnung und der besagte Flüssigkeitseintrittsdurchgang (520) durch den besagten Flüssigkeitsströmungspfad untereinander verbunden sind und das besagte Einfüllmittel ein Eintrittskontrollventil für die Steuerung der in den besagten Flüssigkeitseintrittsdurchgang strömenden Flüssigkeit enthält.

22. Vorrichtung nach Anspruch 19, bei der die besagten Dosiermittel einen Zusatzstoff und ein Darstellungsmittel für den Zusatzstoff zur Darstellung des besagten Zusatzstoffes in der Flüssigkeit enthalten, wobei das besagte Darstellungsmittel für Zusatzstoffe innerhalb des Flüssigkeitseintrittsdurchgangs (520) zwischen der besagten Flüssigkeitsaustrittsöffnung und dem besagten Einfüllkontrollventil angeordnet ist.

23. Vorrichtung nach Anspruch 19, bei der die besagten Dosiermittel einen Zusatzstoff und ein Darstellungsmittel für den Zusatzstoff zur Darstellung des besagten Zusatzstoffes in der Flüssigkeit enthalten, wobei der besagte Zusatzstoff von dem besagten Darstellungsmittel für Zusatzstoffe unter Benutzung der Technik der Affinitätschromatographie entfernbar ist.

24. Vorrichtung nach Anspruch 23, bei der der besagte Zusatzstoff mit dem besagten Darstellungsmittel für Zusatzstoffe unter Benutzung von funktionellen Azlactonverbindungen entfernbar verbunden ist.

25. Vorrichtung nach Anspruch 23, bei der ein Ligand mit dem besagten Darstellungsmittel für Zusatzstoffe verbunden ist und ein Target-Molekül mit dem besagten Liganden verbunden ist.

26. Vorrichtung nach Anspruch 25, bei der ein Abstandsstück mit dem besagten Träger verbunden ist und in der Enzyme mit dem besagten Abstandsstück verbunden sind.

27. Vorrichtung nach Anspruch 25, bei der ein Abstandsstück mit dem besagten Träger verbunden ist und in der ein Protein mit dem besagten Abstandsstück verbunden ist.

28. Vorrichtung nach Anspruch 27, bei der ein Antikörper an das besagte Protein gebunden ist.

29. Vorrichtung nach Anspruch 7, bei der die besagte Vorrichtung für die Infusion von Flüssigkeiten mit einem gesteuerten Volumenstrom in einen ambulant versorgten Patienten benutzt wird und bei der die besagte Flüssigkeitsaustrittsöffnung (608) ein Flüssigkeitsaustrittsdurchgang und ein Flüssigkeitsdurchgang ist, der die besagte Flüssigkeitseintrittsöffnung und den besagten Flüssigkeitsaustrittsdurchgang untereinander verbindet, wodurch die besagte Kammer (620) in Verbindung mit dem besagten Flüssigkeitsdurchgang ist und worin die besagten Dosiermittel innerhalb des besagten Flüssigkeitsaustrittsdurchgangs für die Dosierung eines Zusatzstoffes zur hindurch fließenden Flüssigkeit angeordnet ist und die besagten Dosiermittel einen Träger und einen Zusatzstoff enthalten, der mit dem besagten Träger entfernbar verbunden ist.

30. Vorrichtung nach Anspruch 29, bei der das besagte Einfüilmittel eine Spritzenanordnung enthält.

31. Vorrichtung nach Anspruch 29, bei der ein aufrechter Teil (626) die besagten Dosiermittel, die sich quer zum Grundplattenteil (601) der Vorrichtung erstrecken, aufnimmt.

32. Vorrichtung nach Anspruch 29, bei der der besagte aufrechte Teil (626) sich in Längsrichtung zum besagten Grundplattenteil (601) erstreckt.

## Revendications

1. Dispositif destiné à l'administration contrôlée de liquides médicinaux en perfusion à un patient, comprenant:
(a) une base (300) d'une certaine largeur
(b) et des moyens de distribution associés de manière opérationnelle avec une sortie de liquide (310) pour la distribution de liquide à partir du dispositif,
(c) des moyens d'accumulation de l'énergie formant, en conjonction avec ladite base, une chambre de liquide (211) de faible hauteur comportant une sortie de liquide (213), **caractérisé en ce que**
(d) lesdits moyens d'accumulation de l'énergie comprennent une construction multicouche (318) comportant au moins deux membranes extensibles distinctes (320, 322) superposées sur ladite base (300), lesdites membranes extensibles pouvant être distendues par le liquide à injecter, ce qui crée des contraintes internes tendant à ramener lesdits éléments à une configuration moins distendue, lesdites membranes extensibles ayant une action propre à pousser le liquide hors de ladite chambre à travers ladite sortie.

2. Dispositif selon la revendication 1, **caractérisé en ce qu'**il comporte en outre un moyen de contrôle du débit disposé à l'intérieur de ladite chambre afin de contrôler le débit d'écoulement du liquide hors dudit dispositif, ce moyen de contrôle du débit interne comprenant au moins une membrane perméable mince superposée sur ladite base.

3. Dispositif selon les revendications 1 et 2, **caractérisé en ce qu'**il comporte en outre un moyen de contrôle du débit externe disposé entre ladite sortie de liquide et lesdits moyens de distribution en vue de contrôler le débit d'écoulement du liquide hors dudit dispositif.

4. Dispositif selon l'une quelconque des revendications 1 à 3,
**caractérisé en ce que** l'une au moins desdites membranes extensibles planes est perméable au gaz.

5. Dispositif selon la revendication 4, **caractérisé en ce que** l'une au moins desdites membranes extensibles a une première constante de perméabilité.

6. Dispositif selon la revendication 5, **caractérisé en ce que** l'une au moins desdites membranes extensibles a une deuxième constante de perméabilité.

7. Dispositif selon la revendication 1 servant à injecter des liquides en perfusion à un patient ambulatoire à un débit contrôlé **caractérisé en ce que**,
(a) ladite entrée de liquide et ladite sortie de liquide (310) de ladite base sont reliées entre elles par un trajet de circulation du liquide ;
(b) ladite membrane extensible (320, 322) est faite d'un matériau élastique ajusté par-dessus ladite base (300) afin de définir ladite chambre et communique avec ladite entrée de liquide et ladite sortie de liquide (310), lequel dispositif comporte en outre :
(c) des moyens de remplissage pour introduire du liquide dans ladite entrée de liquide et
(d) des moyens d'addition disposés dans ledit trajet de circulation du liquide pour ajouter un additif au liquide qui y circule.

8. Dispositif selon la revendication 7, **caractérisé en ce que** ledit moyen d'addition comprend un additif et un moyen de présentation de l'additif destiné à présenter ledit additif au liquide, ledit moyen de présentation de l'additif étant disposé dans ledit trajet de circulation de telle sorte qu'une partie au moins dudit additif soit ajoutée audit liquide introduit dans ladite entrée de liquide.

9. Dispositif selon la revendication 7, **caractérisé en ce que** ledit moyen d'addition comprend un additif et un moyen de présentation de l'additif destiné à présenter ledit additif dans le liquide, ledit moyen de présentation de l'additif comportant des surfaces exposées et ledit additif étant relié de manière amovible auxdites surfaces exposées.

10. Dispositif selon la revendication 7, **caractérisé en ce que** le moyen d'addition comprend un support structurel possédant des surfaces exposées et un agent thérapeutique présent sur lesdites surfaces exposées, ledit support structurel étant disposé dans ledit trajet de circulation du liquide de telle sorte qu'une partie au moins dudit agent thérapeutique soit ajoutée au liquide introduit dans ladite entrée de liquide.

11. Dispositif selon la revendication 7, **caractérisé en ce que** ledit moyen d'addition comporte un support en polymère et un additif porté par ledit support en polymère, lequel support en polymère est disposé dans ledit trajet de circulation du liquide de telle sorte qu'une partie au moins dudit additif soit ajoutée au liquide circulant dans le trajet de circulation du liquide.

12. Dispositif selon la revendication 7, **caractérisé en ce que** ledit moyen d'addition comprend un élément possédant des surfaces exposées et un matériau ayant une activité biologique présent sur lesdites surfaces exposées, ledit élément étant disposé dans le trajet de circulation du liquide de telle sorte que ledit matériau ayant une activité biologique soit ajouté au liquide introduit dans ladite entrée de liquide.

13. Dispositif selon la revendication 7 servant à injecter des liquides en perfusion à un patient à un débit contrôlé et **caractérisé en ce qu'**il comporte en outre:
un passage de liquide (420) reliant ladite entrée et ladite sortie de liquide (310), de telle sorte que ladite chambre communique avec ledit passage de liquide (420) et le liquide est introduit dans ladite chambre sous pression en passant par ledit passage de liquide (420),
et dans lequel ledit moyen d'addition est monté dans la dite base et comprend un moyen de présentation de l'additif disposé dans ledit passage du liquide en vue de présenter un additif au liquide qui y circule, et un additif relié de manière amovible audit moyen de présentation de l'additif et contenant un agent thérapeutique.

14. Dispositif selon la revendication 11, **caractérisé en ce que** ladite membrane extensible possède une surface en contact avec le liquide introduit par ledit moyen de remplissage et ledit additif est relié de manière amovible avec cette surface.

15. Dispositif selon la revendication 13, **caractérisé en ce que** ledit moyen de présentation de l'additif comprend une membrane de contrôle du débit disposée entre ladite sortie de liquide de ladite base et ladite chambre, cette membrane de contrôle du débit possédant des surfaces exposées et l'additif étant relié de manière amovible à ces surfaces exposées.

16. Dispositif selon la revendication 13, **caractérisé en ce que** ledit moyen de présentation de l'additif comprend une surface dans ladite base, ledit additif étant relié de manière amovible à cette surface.

17. Dispositif selon la revendication 13, **caractérisé en ce que** ledit moyen d'addition comprend un ensemble de flacon pouvant être obturé et contenant ledit moyen de présentation de l'additif.

18. Dispositif selon la revendication 17, **caractérisé en ce que** ledit ensemble de flacon comprend un flacon de verre dont la première et la seconde extrémité sont obturées par un premier et un second obturateurs.

19. Dispositif selon la revendication 18, **caractérisé en ce que** ladite base comprend une chambre destinée à recevoir ledit flacon de verre.

20. Dispositif selon la revendication 19, **caractérisé en ce qu'**une aiguille creuse est montée dans ladite chambre (211) pour percer ledit second obturateur, l'aiguille creuse possédant un passage de liquide (520) qui communique avec ledit passage de liquide de la base.

21. Dispositif selon la revendication 7 disposé de manière à injecter des liquides en perfusion à un patient ambulatoire, **caractérisé en ce que** ladite base (501) possède une partie verticale (519) comprenant un passage d'entrée de liquide (520), ladite sortie de liquide et ledit passage d'entrée de liquide (520) étant reliés audit trajet de circulation du liquide et ledit moyen de remplissage contenant une valve d'arrêt du remplissage destinée à contrôler l'écoulement dans ledit passage d'entrée du liquide.

22. Dispositif selon la revendication 19, **caractérisé en ce que** ledit moyen d'addition comprend un additif et un moyen de présentation de l'additif destiné à présenter ledit additif au liquide, le moyen de présentation de l'additif étant disposé dans ledit passage d'entrée de liquide (520) entre ladite sortie et ladite vanne d'arrêt du remplissage.

23. Dispositif selon la revendication 19, **caractérisé en ce que** ledit moyen d'addition comprend un additif et un moyen de présentation de l'additif destiné à présenter ledit additif au liquide, ledit additif pouvant être enlevé dudit moyen de présentation de l'additif par des techniques de chromatographie d'affinité.

24. Dispositif selon la revendication 23, **caractérisé en ce que** ledit additif est relié de manière amovible audit moyen de présentation de l'additif par des composés fonctionnels d'azlactone.

25. Dispositif selon la revendication 23, **caractérisé en ce qu'**un ligand est relié audit moyen de présentation de l'additif et une molécule cible est reliée audit ligand.

26. Dispositif selon la revendication 25, **caractérisé en ce qu'**une entretoise est reliée audit support et des enzymes sont reliées à ladite entretoise.

27. Dispositif selon la revendication 26, **caractérisé en ce qu'**une entretoise est reliée audit support et une protéine est reliée à ladite entretoise.

28. Dispositif selon la revendication 27, **caractérisé en ce qu'**un anticorps est lié à ladite protéine.

29. Dispositif selon la revendication 7 servant à injecter des liquides en perfusion à un patient ambulatoire à un débit contrôlé, **caractérisé en ce que** la sortie de liquide (608) est un passage de sortie de liquide et un passage de liquide relie ladite entrée de liquide et ledit passage de sortie de liquide, ce qui met ladite chambre (620) en communication avec ledit passage de liquide, et **en ce que** ledit moyen d'addition est disposé dans ledit passage de sortie de liquide de manière à ajouter un additif au liquide qui y circule, ledit moyen d'addition comprenant un support et un additif relié de manière amovible à celui-ci.

30. Dispositif selon la revendication 29, **caractérisé en ce que** ledit moyen de remplissage comprend un ensemble de seringue.

31. Dispositif selon la revendication 29, **caractérisé en ce qu'**une partie verticale (626) recevant ledit moyen d'addition s'étend transversalement par rapport à l'élément de base (601) du dispositif.

32. Dispositif selon la revendication 29, **caractérisé en ce que** ladite partie verticale (626) s'étend longitudinalement par rapport à l'élément de base (601).
